# EUROPÄISCHE PATENTSCHRIFT

(11) **EP 2 298 084 B1**
(45) Veröffentlichungstag und Bekanntmachung des Hinweises auf die Patenterteilung: **19.10.2011**
(21) Anmeldenummer: 09168973.7
(22) Anmeldetag: 28.08.2009
(51) Int. Cl.: A23L 1/22, A23L 1/236, A23L 1/30, A23L 2/60, A61K 8/49, A61Q 11/00

(54) **Süßmittelreduzierte Produkte, Aromamischungen dafür sowie Verfahren zum Herstellen solcher Produkte**
Sweetener-reduced products, aromatic mixtures for same and method of producing such products
Produits réduits en saccharine, mélanges d'arômes correspondant et procédé de fabrication de tels produits

(43) Veröffentlichungstag der Anmeldung: 23.03.2011
(73) Patentinhaber: Symrise AG, 37603 Holzminden (DE)
(72) Erfinder: Reichelt, Katharina, 83026, Rosenheim (DE); Ley, Jakob, 37603, Holzminden (DE); Hoffmann-Lücke, Petra, 31073, Delligsen (DE); Blings, Maria, 37603, Holzminden (DE); Paetz, Susanne, 37671, Höxter (DE); Riess, Thomas, 37603, Holzminden (DE)
(74) Vertreter: Eisenführ, Speiser & Partner

(56) Entgegenhaltungen:
- WO-A-03/103415
- WO-A-2005/020721
- JP-A- 11 253 128
- JP-A- 2004 256 403
- US-A1- 2007 116 829
- US-A1- 2008 305 052

## Beschreibung

Die Erfindung betrifft eine Aromamischung umfassend einen oder mehrere süß schmeckende Stoffe sowie Phyllodulcin. Diese Aromamischung ist geeignet als Zwischenprodukt für ein oral konsumierbares Produkt, enthaltend eine solche Aromamischung, wobei in dem oral konsumierbaren Produkt der Anteil an üblichen süß schmeckenden Stoffen reduziert ist. Die Erfindung betrifft ferner ein Verfahren zur Herstellung eines süßmittelreduzierten oral konsumierbaren Produktes sowie die Verwendung einer entsprechenden Aromamischung zur Herstellung eines solchen Produktes.

Nahrungs- oder Genussmittel sowie Getränke und andere oral konsumierbare Produkte, die einen hohen Zuckergehalt (vor allem Sucrose (= Saccharose), Lactose, Glucose oder Fructose oder deren Mischungen) aufweisen, werden in der Regel von Verbrauchern auf Grund der Süße stark präferiert. Auf der anderen Seite ist allgemein bekannt, dass ein hoher Gehalt an leicht verstoffwechselbaren Kohlenhydraten den Blutzuckerspiegel stark ansteigen lässt, zur Bildung von Fettdepots führt und letztendlich zu gesundheitlichen Problemen wie Übergewicht, Fettleibigkeit, Insulinresistenz, Altersdiabetes und deren Folgeerkrankungen führen kann. Insbesondere kommt erschwerend hinzu, dass viele der oben genannten Kohlenhydrate zusätzlich die Zahngesundheit beeinträchtigen können, da sie von bestimmten Bakteriensorten in der Mundhöhle zu beispielsweise Milchsäure abgebaut werden und den Zahnschmelz der juvenilen oder adulten Zähne angreifen können (Karies).

Daher ist es schon lange ein Ziel, den Zuckergehalt von Nahrungs- und/oder Genussmitteln und Getränken soweit wie möglich zu reduzieren, wobei bevorzugtes Ziel ist, diese Reduktion mit möglichst geringer Verringerung des Süßeindruckes zu erreichen. Eine entsprechende Maßnahme besteht im Einsatz von Süßstoffen: das sind chemisch einheitliche Substanzen, die selbst keinen oder nur einen sehr geringen kalorischen Brennwert haben und gleichzeitig einen starken süßen Geschmackseindruck verursachen; die Stoffe sind in der Regel nicht-kariogen (eine Übersicht ist beispielsweise zu finden in Journal of the American Dietetic Association 2004, 104 (2), 255-275). Die nicht-nutritiven, hochintensiven Süßstoffe sind zwar durch ihre geringe Einsatzkonzentration gut geeignet, Süße in Nahrungsmittel zu bringen, zeigen jedoch oft geschmackliche Probleme durch im Vergleich zu Zucker unähnliche Zeit-Intensitätsprofile (beispielsweise Sucralose, Stevia-Produkte, Rebaudioside, Stevioside, Cyclamat), einen bitteren und/oder adstringierenden Nachgeschmack (beispielsweise Acesulfam K, Saccharin, Stevia-Produkte, Rebaudioside, Stevioside), ausgeprägte zusätzliche Aromaeindrücke (beispielsweise Glycerrhyzinsäureammoniumsalz). Einige der Süßstoffe sind gegenüber Hitze nicht besonders stabil (beispielsweise Thaumatin, Brazzein, Monellin), sind nicht in allen Anwendungen stabil (beispielsweise Aspartam) und sind teilweise sehr langanhaltend in ihrer süßen Wirkung (starker süßer Nachgeschmack, beispielsweise Saccharin, Stevia-Produkte, Rebaudioside, Stevioside).

Eine andere Möglichkeit - ohne den Einsatz von nicht-nutritiven Süßstoffen - besteht darin, den Zuckergehalt von Nahrungs- und/oder Genussmitteln zu senken und sensorisch schwach oder nicht wahrnehmbare Stoffe zuzusetzen, die die Süße mittelbar oder unmittelbar verstärken, wie z.B. in WO 2005/041684 beschrieben. Die in WO 2005/041684 beschriebenen Stoffe sind aber ausdrücklich nicht-natürlichen Ursprungs und somit aus toxikologischer Sicht schwieriger zu bewerten als Stoffe natürlicher Herkunft, insbesondere wenn letztere in Nahrungs- oder Genussmitteln vorkommen oder aus Rohstoffen zur Nahrungs- oder Genussmittelgewinnung stammen. In EP 1 291 342 werden solche Stoffe natürlicher Herkunft (Pyridinium-Betaine) beschrieben; allerdings wird durch sie nicht selektiv der süße Geschmack, sondern auch andere Geschmacksrichtungen wie Umami oder Salzigkeit beeinflusst. Zudem sind die offenbarten Stoffe nur mit hohem Aufwand zu reinigen.

In der WO 2007/014879 A1 wird der Einsatz von Hesperetin als Verstärker des süßen Geschmacks von Zucker-reduzierten, der Ernährung oder dem Genuss dienenden Zubereitungen empfohlen. Bisweilen nachteilig ist allerdings bei Einsatz von Hesperetin dessen geringe Löslichkeit insbesondere in klaren, wässrigen Anwendungen (wie z.B. klaren Cola- oder Zitronenlimonaden) sowie die vergleichsweise schwach ausgeprägte Süßverstärkung in sauren Nahrungs- und Genussmitteln. In der WO 2007/014879 A1 wird die Verwendung von Hesperetin auch in Kombination mit 4-Hydroxydihydrochalkonen beschrieben. Dabei können schon erhebliche Reduzierungen der Zucker- oder Süßstoff mengen bei gleicher Süße und weitgehend ausreichender Löslichkeit erreicht werden, allerdings in vielen Fällen keine Wiederherstellung der Süße bei mehr als 25 % Reduktion der Zucker- oder Süßstoffmengen vor allem in stärker sauren (pH < 5) Applikationen.

Das in Hydrangea dulcis-Blättern vorkommende Isocumarin Phyllodulcin ist schon als Süßstoff beschrieben worden (Kinghorn, A. D. and C. M. Compadre (2001). "Less common high-potency sweeteners." Food Sci. Technol. (N.Y.) 112(Alternative Sweeteners): 209-233, Crosby, G. A. (1976). "New Sweeteners." Critical Reviews in Food Science and Nutrition(June): 297-323). Die Verwendung als *synergistisch* wirkender Aromastoff insbesondere unterhalb der eigentlich alleine süß schmeckenden Wirkung ist allerdings noch nie beschrieben worden.

In JP 11253128 wird zwar schon eine synergistische Kombination von Euonymus tricocarpus mit Hydrangea dulcis Extrakten beschrieben. Aus dem Text geht aber hervor, dass als Wirkung hier der antioxidative Effekt im Vordergrund steht.

In WO 2005/020721 wird ein Getränk mit einer Kombination eines Süßstoffes, Monatin, mit Phyllodulcin als isoliertem Süßstoff beansprucht. Jedoch ist hier die Konzentration an Phyllodulcin ausreichend, um einen süßen Geschmack auch alleine zu verursachen.

Die WO 2007/121604 beschreibt ein Verfahren zur Identifikation von Modulatoren der T1 R2-TMD und/oder CFR:T1R-Rezeptoren. In einer großen Liste für potentielle Testkandidaten ist auch Phyllodulcin aufgeführt. Es gibt in dem Dokument allerdings keinen ausreichenden Hinweis darauf, dass Phyllodulcin ein Enhancer der zu testenden Rezeptorsysteme sein könnte, da diese Verbindung lediglich als potentieller Modulator bebeschrieben wurde. Insbesondere enthält das Dokument keinen Hinweis, dass Phyllodulcin *in vivo* einen synergistisch verstärkenden Effekt auf die Süßwahmehmung haben könnte. Erst recht enthält das genannte Dokument keinen Hinweis darauf, in welchen Verhältnissen Phyllodulcin und eventuell zu verstärkende SüßmMel *in vitro* oder *in vivo* eingesetzt werden sollen.

In JP 2004 256403 A beschreibt Phyllodulcin als Additiv unter anderem für Nahrungsmittel, wobei Phyllodulcin die Funktion haben soll, die Produktion von TNF-alpha zu inhibieren. Der Einsatz von Phyllodulcin erfolgt in verhältnismäßig hohen Konzentrationen (z.B. 0,3 Gew.-%).

In US 2008/305052 A1 wird eine Kombination von Hesperetin mit z.B. Phyllodulcin beschrieben. Jedoch erfolgt kein Hinweis darauf, in welchen Verhältnissen Hesperetin und Phyllodulcin eingesetzt werden sollen. Im Vordergrund steht hier das Vermögen des Hesperetins, den süßen Geschmack eines süß, schmeckenden Stoffes zu steigem.Die WO 03/103415 A1 beschreibt eine Saßstoffkomposition, die Kiwifrucht, Fruchtzucker und ein Fruchtglykosid enthält wobei das Fruchtglykosid Phyllodulcin sein kann. Das Dokument enthält jedoch keinen Hinweis, dass Phyllodulcin einen synergistisch verstärkenden Effekt auf die Süßwahmehmung haben könnte. Erst recht enthält das genannte Dokument keinen Hinweis darauf, in welchem Verhältnis Phyllodulcin eingesetzt werden soll. Der wesentliche Vorteil der in WO 031103415 A1 beschriebenen Kompositionen besteht darin, dass sie die Lipoprotein-Lipase *in vivo* nicht stimulieren.

In US 200710116829 A1 werden pharmazeutische Kompositionen beschrieben, die einen hochpotenten Süßstoff (z.B. Phyllodulcin) und eine weitere Komposition enthalten, wobei die weitere Komposition einen süßen Geschmackseindruck verbessert. Dabei kann die weitere Komposition z.B. Xylitol, also einen selbst süß schmeckenden Stoff, enthalten. Das Dokument trifft keine Aussage Ober das Sucrose-Äquivalenz-Verhältnis des hochpotenten Süßstoffes und des zweiten süß schmeckenden Stoffes zueinander,

Aufgabe der Erfindung war es vor dem Hintergrund des Standes der Technik oral konsumierbare, süß schmeckende Produkte anzugeben, die gegenüber Produkten mit einem vergleichbaren Süßeindruck eine reduzierte Zucker- und/oder Süßstoffmenge umfassen. Bestandteil dieser Aufgabe war es, entsprechende Vorprodukte oder Zwischenprodukte in Form von Aromamischungen anzugeben, die in den oral konsumierbaren süß schmeckenden Produkten in verdünnter Form eingesetzt werden können und den gewünschten Effekt gewahneisten. Bevorzugt sollten die Produkte bzw. Aromakompositionen hinsichtlich des von ihnen vermittetten Süßeindruckes stabil sein, der Süßeindruck auf natürliche vorkommenden Verbindungen basieren und/oder die Reduktion der Zucker- oder Süßestoffmenge bei gleichbleibendem Süßeindruck möglich sein, ohne dass das übliche Aromaprofil negativ beeinflusst wird. Außerdem war enwünscht, dass die Mittel, die eine Reduktion der zucker oder Süßstoffmenge ermöglichen, gut und klar in Wasser löslich sind.

Diese Aufgabe wird hinsichtlich des Vorproduktes bzw- der Aromamischung gelöst durch eine Aromamischung, umfassend
(i) einen oder mehrere süß schmeckende Stoffe, ausgewählt aus der Gruppe der natüdich vorkommenden süß schmeckende Stoffe und deren physiologisch verträgliehen Salzen ohne Phyllodulcin und dessen physiologisch verträglichen Salzen,
   und
(ii) Phyllodulcin und/oder eines oder mehrere dessen physiologisch verträgliche Salze,
wobei das Verhältnis des Sucroseaquivalenz der Konzentration des Stoffes oder der Stoffe der Gruppe (i) zur Sucroseäquivalenz der Konzentration des Stoffes oder der Stoffe der Gruppe (i) ≥ 2, bevorzugt ≥ 3, weiter bevorzugt ≥ 4 und besonders bevorzugt ≥ 6 ist

Der oder die süß schmeckenden Stoffe (Stoffe der Gruppe (i)) sind bevorzugt ausgewählt aus der Gruppe bestehend aus
a) Kohlenhydraten, bevorzugt ausgewählt aus der Untergruppe bestehend aus Sucrose, Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd und Maltodextrin;
b) natürlich vorkommenden Zuckeralkoholen ausgewählt aus der Untergruppe bestehend aus Glycerin, Erythritol, Threitol, Arabitol, Ribitol, Xylitol, Sorbitol, Mannitol, Maltitol, Isomaltit, Dulcitol und Lactitol. Die Kohlenhydrate können auch in Form von pflanzlichen Zubereitungen enthaltend eine oder mehrere der genannten Kohlenhydrate, vorzugsweise in einem Anteil von zumindest 5 Gew.-%, bevorzugt zumindest 15 Gew,-%) vorliegen. Die Kohlenhydrate können auch natürlich vorkommende oder künstlich hergestellte Mischung vorliegen (z.B. aus Honig, Invertzucker, Sirup, hochangereichtere Fructose-Sirup aus Maisstärke (High Fructose Corn Syrup);
c) natürlich vorkommenden Süßstoffen, ausgewählt aus der Untergruppe bestehend aus Miraculin, Curculin, Monellin, Mabinlin, Thaumatin, Curculin, Brazzein, Pentadin, D-Phenylalanin und D-Tryptophan;
d) natürlich vorkommenden Süßstoffen, ausgewählt aus der Untergruppe bestehend aus Steviosid, Steviolbiosid, Rebaudiosid A, weiteren Steviolglycosiden wie Rebaudiosid B, Rebaudiosid C, Rebaudiosid D, Rebaudiosid E, Rebaudiosid F, Rebaudiosid G, Rebaudiosid H, Dulcosid und/oder Rubusosid, Oslandin, Polypodosid A, Strogin 1, Strogin 2, Strogin 4, Selligueanin A, Dihydroquercetin-3-acetat, Perillartin, Telosmosid A₁₅, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Hernandulcin, Glycyrrhetinsäure;
e) den physiologisch verträglichen, süß schmeckenden Derivaten der Mitglieder der Untergruppen a) bis d); und
f) den physiologisch verträglichen Salzen der Mitglieder der Untergruppen a) bis e), insbesondere deren Kalium-, Natrium-, Calcium- oder Ammoniumsalze.

Die in der Untergruppe d) genannten Süßstoffe können bevorzugt in Form von aus natürlichen Quellen gewonnenen Extrakten oder Fraktionen, enthaltend die süß schmeckenden Aminosäuren und/oder Proteine vorliegen.

Natürlich vorkommende Süßstoffe im Sinne der Erfindung sind nicht-nutritive oder nur in geringem Maße nutritive, vor allem süß schmeckende Verbindungen oder vor allem süß schmeckende natürliche Extrakte oder Isolate, wobei diese natürlichen Extrakte oder Isolate nicht die süß schmeckenden Kohlenhydrate, insbesondere Zucker oder Zuckeralkohole, in einer Menge umfassen, die ausreicht, um einen wahrnehmbaren süßen Geschmack in der Zubereitung auszulösen. Dabei müssen die Süßstoffe im Sinne der Erfindung alleine in der Lage sein, eine Süßkraft in der zu konsumierenden Zubereitung (Lebens-, Genussmittel) zu erzeugen, die der Süßkraft einer mindesten 4 %igen wässrigen Sucrose-Lösung entsprächen.

Die natürlich vorkommenden Süßstoffe aus der Gruppe (i) können in der erfindungsgemäßen Aromamischungen auch eingesetzt werden in Form von Extrakten oder angereicherten Fraktionen dieser Extrakte, insbesondere Thaumatococcus-Extrakte (Katemfestaude), Extrakte aus *Stevia* ssp. (insbesondere *Stevia rebaudiana*), Swingle-Extrakt (*Momordica* bzw. *Siratia grosvenorii,* Luo-Han-Guo), Extrakte aus Süßholzwurzel, auch *Glycerrhyzia* ssp. (insb. *Glycerrhyzia glabra*), *Rubus* ssp. (insbesondere *Rubus suavissi*mus), Citrus-Extrakte, Extrakte aus *Lippia dulcis* entsprechend angereicherte Fraktionen dieser Extrakte.

Das Sucrose-Äquivalenz-Verhältnis der Sucrose-Äquivalenz der Konzentration der Stoffe oder des Stoffes der Gruppe (i) zu der Sucrose-Äquivalenz der Konzentration des Stoffes oder der Stoffe der Gruppe (ii) in der entsprechenden Aromamischung (und in anderen Mischungen, vgl. weiter unten) wird wie folgt festgestellt:
1. Es wird eine Vergleichsreihe verschiedener Sucrose-Konzentrationen in Wasser hergestellt. Bevorzugte Konzentrationen sind 0; 0,25; 0,5; 0,75; 1; 1,5; 2; 3; 4 und 5 Gew.-% Sucrose in Wasser. Gegebenenfalls können auch andere Konzentrationen, insbesondere höhere Konzentrationen für die Vergleichsreihe eingesetzt werden.
2. Es wird eine wässrige Lösung der süß schmeckenden Stoffe der Gruppe (i) mit einer gleichen Konzentration dieser Stoffe wie in der zu untersuchenden Probe hergestellt (Lösung 1).
3. Es wird eine wässrige Lösung der Stoffe der Gruppe (ii) mit der gleichen Konzentration wie die der Stoffe der Gruppe (ii) in der zu untersuchenden Probe hergestellt (Lösung 2).
4. Ein Panel von mindestens 10 Personen (bevorzugt Personen, die im Bereich des Abschmeckens über Erfahrung verfügen, verschiedenen Alters, Herkunft und Geschlechts) testet die Lösung 1 gegenüber der Sucrose-Reihe und ordnet sie ein, um die Sucrose-Äquivalenz zu bestimmen.
5. Die ermittelte Sucrose-Äquivalenz ergibt sich aus dem Mittelwert der Einzeleinstufungen der einzelnen Panelisten.
6. Nun wird das Verdünnungsverhältnis bestimmt, mittels dessen die Lösung 1 auf eine Sucrose-Äquivalenz von 2 Gew.-% Sucrose in Wasser eingestellt werden kann. Dafür kann es sinnvoll sein, die Testprobe bereits vor dem Schritt 4 vorzuverdünnen. Selbstverständlich ist es sinnvoll, das ermittelte Verdünnungsverhältnis gegebenenfalls noch einmal durch die Panelisten prüfen zu lassen.
7. Nun wird die Lösung 2 (enthaltend die Verbindung(en) der Gruppe (ii)) mit dem unter 6. ermittelten Verdünnungsverhältnis verdünnt.
8. Nachfolgend wird die verdünnte Lösung 2 ebenfalls gegen die Sucrose-Testreihe abgeschmeckt, von den Panelisten eingeordnet und ein entsprechender Sucrose-Äquivalenz-Wert bestimmt (analog zu den Punkten 4. und 5.)
9. Die Sucrose-Äquivalenz der verdünnten Lösung 1 (äquivalent zu 2 Gew.-% Sucrose in Wasser) wird in das Verhältnis zu der gemäß Schritt 8. bestimmten Sucrose-Äquivalenz der verdünnten Lösung 2 gesetzt. Dies ergibt das Sucrose-Äquivalenz-Verhältnis.

Der Vorteil der erfindungsgemäßen Aromamischung besteht insbesondere darin, dass sie als Vorprodukt (oder Zwischenprodukt) für oral konsumierbare Produkte besonders gut zu verwenden ist: Das eingestellte Sucrose-Äquivalenz-Verhältnis der Stoffe der Gruppen (i) und (ii) ermöglicht eine synergistische Verstärkung des Süßeindruckes. Hierbei ist zu beachten, dass die Stoffe der Gruppe (ii) (Phyllodulcin und dessen physiologisch vertretene Salze) bereits in extrem geringen Konzentrationen eine sehr stark ausgeprägte Süßkraft (und damit hohe Sucrose-Äquivalenz) besitzen. Dies bedeutet, dass in den erfindungsgemäßen Aromamischungen die Konzentration der Stoffe der Gruppe (i) regelmäßig um ein Vielfaches höher ist als die Konzentration der Stoffe der Gruppe (ii).

Der durch die erfindungsgemäße Aromamischung bzw. durch deren Verdünnungen erzeugbare Süßeindruck ist dabei deutlich höher als der (z.B. durch Sucrose-Äquivalenz messbare) Süßeindruck der Verbindungen der Gruppe (i) und der Gruppe (ii) miteinander addiert. Es liegt somit ein überraschender synergistischer Effekt vor. Dabei ist dieser synergistische Effekt überraschenderweise sowohl im Bereich unterhalb als auch im Bereich oberhalb der Wahrnehmungsschwelle von Phyllodulcin zu beobachten (vgl. auch weiter unten).

Erfindungsgemäß bevorzugt ist eine erfindungsgemäße Aromamischung, umfassend einen Extrakt aus *Hydrangea dulcis* als Quelle für die Verbindung oder die Verbindung der Gruppe (ii).

Im Sinne dieses Textes zählen zu "*Hydrangea dulcis*" alle Hydrangea-Variationen, die Phyllodulcin enthalten. Bevorzugte *Hydrangea dulcis*-Variationen sind *Hydrangea macrophylla* var. thunbergii, *Hydrangea macrophylla* var. oamacha und *Hydrangea macrophylla var.* Amagiana und weiterer, natürlicherweise Phyllodulcin enthaltende Hydrangea-Species.

In einigen Fällen kann es bevorzugt sein, dass der erfindungsgemäß bevorzugt enthaltene Extrakt kein wässriger Extrakt ist, das heißt, kein Extrakt, der ausschließlich unter Einsatz von Wasser als Extraktionsmittel erzeugt wurde.

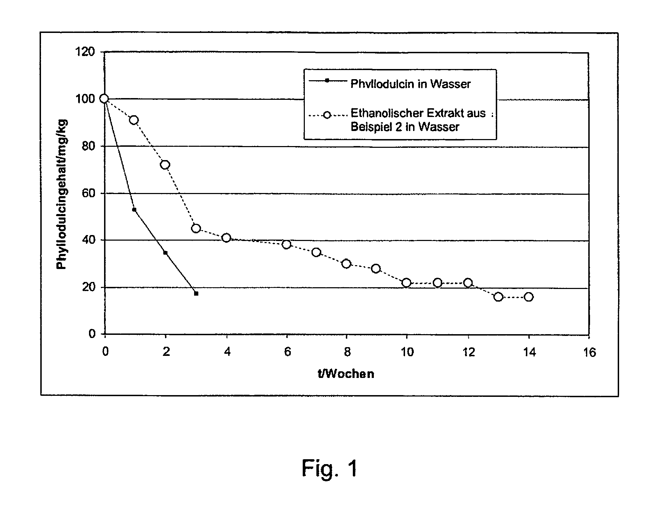

Vorteil des Einsatzes solcher Extrakte liegt darin, dass überraschenderweise das Phyllodulcin stabiler ist, das heißt auch unter für den Lebensmittelbereich verhältnismäßig rigiden Bedingungen eine langsamere Degeneration zeigt. Hierzu sei auch auf die Figur 1 und das Beispiel 2 (vgl. weiter unten) verwiesen.

So konnte in einer wässrigen Lösung eines beispielhaften Extraktes eine dreifach höhere Stabilität bei gleicher absoluter Phyllodulcin-Anfangskonzentration beobachtet werden.

Bevorzugt lässt sich das Vorliegen (oder das Enthalten sein) eines Extraktes aus *Hydrangea dulcis* (oder den bevorzugten Hydrangea-Variationen) durch Vorliegen der Verbindung Hydrangenol belegen.

Das Hydrangenol kann auch in der offenen Form als Hydrangea-Säure vorliegen.

Zur Verdeutlichung sind die Strukturen der wichtigen Inhaltsstoffe des Extraktes in der folgenden Abbildung zusammengefasst: Phyllodulcin (1), (2S)-Phyllodulcin (S-1), Hydrangenol (2), (2S)-Hydrangenol (S-2), E- und Z-Hydrangeasäure (E-3, Z-3).

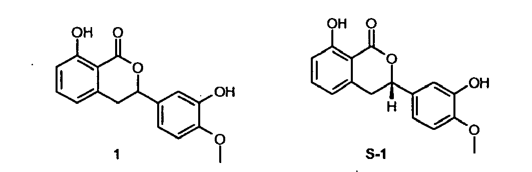

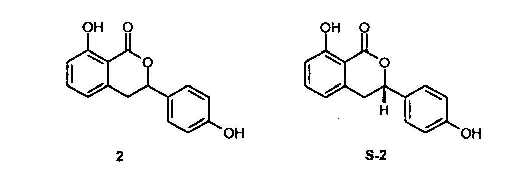

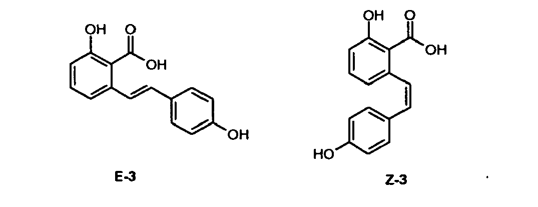

Für den erfindungsgemäß bevorzugt einzusetzenden Extrakt aus *Hydrangea dulcis* (wiederum bevorzugt *Hydrangea macrophylla var. thunbergii*) ist es bevorzugt, dass er mindestens 0,1 Gew.-% und höchstens 50 Gew.%, bevorzugt 0,5 Gew.-% bis 30 Gew.-%, besonders bevorzugt 1 Gew.% bis 25 Gew.-% Phyllodulcin und mindestens 0,01 % und höchstens 50 %, bevorzugt 0,01 Gew.-% bis höchstens 30 Gew.-%, besonders bevorzugt 0,01 Gew.-% bis höchstens 25 Gew.-%, Hydrangenol, jeweils bezogen auf das Trockengewicht des Extrakts enthält.

Die bevorzugt einzusetzenden Extrakte für die bevorzugt erfindungsgemäße Aromamischung werden bevorzugt dadurch gewonnen, dass man die direkt nach der Ernte getrockneten Blätter der Pflanze
a) mit Wasser anfeuchtet, 1 bis 72 h, bevorzugt 5 bis 16 h feucht bei 10 bis 50°C, bevorzugt 15 bis 25°C fermentiert;
b) die feuchten, fermentierten Blätter mit einem geeigneten Lösungsmittel (z.B. Wasser, sub- oder superkritischem Wasser, superkritischem CO₂, Wasser-Ethanol-Gemische, Ethanol, Essigsäureethylester, n-Heptan, n-Hexan, wobei auch mineralische oder organische Säuren wie Phosphorsäure, Zitronensäure, Äpfelsäure, Bernsteinsäure, Essigsäure, Ameisensäure, Fumarsäure, Maleinsäure oder andere zugelassene Genusssäuren verwendet werden können) von 0°C bis zur Siedetemperatur des jeweiligen Lösungsmittels, unter Normaldruck, reduziertem oder erhöhtem Druck z.B. durch Soxhlet-, Gegenstrom-, Perkolationsoder einfaches Siebkorbverfahren extrahiert (Primärextrakt);
c) den Primärextrakt ggfs. durch destillative oder andere evaporative oder pervaporative Verfahren aufkonzentriert;
d) gegebenenfalls einer Aufreinigung des konzentrierten Primärextraktes durch Behandlung mit Adsorbentien (Kieselgel, Aktivkohle, Kieselgur, Alumina, basische oder saure oder neutrale lonentauscher) im Batch oder Säulenverfahren durchführt (Sekundärextrakt);
e) den Sekundärextrakt durch evaporative oder pervaporative Verfahren trocknet;
f) und fakultativ den getrockneten Sekundärextrakt wieder in geeignete Lösungsmittel oder Gemische aufnimmt (Ethanol, 1,2-Propylenglycol, Pflanzenöltriglyceride, Triacetin, Glycerin); und
g) mit mineralischen oder organische Säuren wie Phosphorsäure, Zitronensäure, Äpfelsäure, Bernsteinsäure, Essigsäure, Ameisensäure, Fumarsäure, Maleinsäure oder andere zugelassene Genusssäuren sauer einstellt.

Evaporative oder pervaporative Verfahren können z.B. Destillation, Sublimation, Wasserdampfdestillation, Gefriertrocknung, pervaporative Membranverfahren oder Sprühtrocknung sein, wobei dazu auch vor dieser Behandlung geeignete Hilfs- und Trägerstoffe zugesetzt werden können.

Dabei wird das Verfahren dergestalt durchgeführt, dass der Extrakt 0,1 Gew.-% und höchstens 50 Gew.-%, bevorzugt 0,5 Gew.-% bis 30 Gew.%, besonders bevorzugt 1 Gew.-% bis 25 Gew.-% Phyllodulcin und höchstens 50 %, bevorzugt 0,01 Gew.-% bis höchstens 30 Gew.-%, besonders bevorzugt 0,01 Gew.% bis höchstens 25 Gew.-%, Hydrangenol enthält.

Eine bevorzugte erfindungsgemäße Aromamischung umfasst des Weiteren einen oder mehrere Stoffe, ausgewählt aus der Gruppe (iii), bestehend aus einen süßen Geschmack verstärkenden Aroma- und/oder Geschmackstoffen und deren physiologisch verträglichen Salzen.

Überraschenderweise hat sich gezeigt, dass sich zusätzlich zu dem durch Phyllodulcin bzw. seine Salze hervorgerufenen synergistischen Effekt der Süßverstärkung ein weiterer synergistischer Effekt mit weiteren aus dem Stand der Technik bekannten Süßverstärkern ergibt.

Besonders bevorzugt sind als weitere Süßverstärker in diesem Zusammenhang:
- Hesperetin nach WO 2007/014879 A1
- Hydroxyphenylalkadione nach WO 2007/003527 A1
- 4-Hydroxychalkone nach WO 2007/107596 A1 und EP 1 972 203
- Propenylphenylglycoside (Chavicolglycoside) nach EP 1 955 601 A1
- Divanilline und/oder
- Hydroxyflavane.

Die erfindungsgemäßen Produkte können gemäß Gruppe (iii) ein oder mehrere Divanilline enthalten, insbesondere solche wie beschrieben in der WO 2004/078302, dabei bevorzugt ist 6,6'-Dihydroxy-5,5'-dimethoxy-biphenyl-3,3'-dicarbaldehyd.

In erfindungsgemäßen Produkte können ein oder mehrere natürlich vorkommende 4-Hydroxydihydrochalcone gemäß Gruppe (iii) enthalten sein, insbesondere solche wie beschrieben in WO 2007/107596 und EP 1 972 203. Bevorzugt sind insbesondere 3-(4-Hydroxyphenyl)-1-(2,4-dihydroxyphenyl)propan-1-on (Davidigenin) und/oder 3-(4-Hydroxyphenyl-1-(2,4,6-trihydroxyphenyl)propan-1-on (Phloretin).

Hesperitin im Sinne eines Bestandteils gemäß Gruppe (iii) einer erfindungsgemäßen Aromamischung kann insbesondere das (2S)-Enantiomer, das (2R)-Enantiomer oder eine Mischung dieser Enantiomere sein, wie insbesondere in der WO 2007/014879 beschrieben.

Einer erfindungsgemäßen Aromamischung können bevorzugt gemäß Gruppe (iii) als Propenylphenylglycosid (Chavicolglycosid) enthalten sein, die alpha- oder beta-Anomere und ganz besonders die beta-Anomere des 1-0-[4-(Propen-2-enyl)phenyl]-D-glucopyranosids (Chavicolglucosid), 1-0-[4-(Propen-2-enyl)phenyl]-6-0-β-D-apiofuranosyl-D-glucopyranosids (Furcatin), 1-0-[4-(Propen-2-enyl)phenyl]-6-0-β-D-rutinosids und/oder des 1-0-[4-(Propen-2-enyl)phenyl]-O-ß-D-xylopyranosyl-(1-6)-ß-D-glucopyranosids (p-Allylphenylprimeverosid, Miyaginin).

Als Hydroxyphenylalkandion ist Isogingerdion-2 bevorzugt.

Als Hydroxyflavan ist 3',7-Dihydroxy-4'-methoxyflavan und/oder (S)-3',7-Dihydroxy-4'-methoxyflavan bevorzugt.

Im Sinne der oben gestellten Aufgaben war Ziel der Erfindung auch ein oral konsumierbares Produkt, umfassend eine erfindungsgemäße Aromamischung. In dem erfindungsgemäßen Produkt ist das Verhältnis der Sucrose-Äquivalenz der Konzentration des Stoffes oder der Stoffe der Gruppe (i) zur Sucrose-Äquivalenz der Konzentration des Stoffes oder der Stoffe der Gruppe (ii) ≥ 2, bevorzugt ≥ 3, weiter bevorzugt ≥ 4 und besonders bevorzugt ≥ 6. In diesem Bereich lässt sich der synergistische Verstärkungseffekt von Phyllodulcin besonders gut ausnutzen, so dass das oral konsumierbare Produkt letztendlich bei gleichbleibender Süße mit einem deutlich verringerten Einsatz an Konzentrationen von Verbindungen der Gruppe (i) erhalten wird.

Gegebenenfalls umfasst das erfindungsgemäß oral konsumierbare Produkt selbstverständlich einen oder mehrere weitere übliche Lebensmittelbestandteile.

Bevorzugt ist in dem oral konsumierbaren Produkt die Konzentration der Aromamischung so eingestellt, dass die Konzentration der Stoffe der Gruppe (ii) 0,5 bis 15 ppm beträgt, bezogen auf das Gewicht des Produktes.

Schon in dieser geringen Konzentration lässt sich der synergistische Effekt von Phyllodulcin und seinen Salzen hinsichtlich der Süßverstärkung ausnutzen. Dies ist angesichts des Standes der Technik überraschend. Darüber hinaus hatte der Einsatz so geringerer Konzentrationen von Phyllodulcin im oral konsumierbaren Produkt (und natürlich besonders im Endprodukt) den Vorteil, dass unangenehme Aromanebennoten des Phyllodulcins nicht oder nicht störend wahrgenommen werden. So war Phyllodulcin bereits als Süßstoff vorbeschrieben (Crosby, G. A. (1976). "New Sweeteners." Critical Reviews in Food Science and Nutrition(June): 297-323.). Dort war allerdings bereits als schwerwiegender Nachteil der starke Nebengeschmack und zudem ein Nachgeschmack beschrieben. Dies führte dazu, dass Phyllodulcin trotz seiner erheblichen Süßwirkung bislang regelmäßig nicht als Süßstoff verwendet wurde.

Bevorzugt ist ein erfindungsgemäßes oral konsumierbares Produkt, wobei die Konzentration der Aromamischung so eingestellt ist, dass die Konzentration der Stoffe der Gruppe (i) alleine eine Sucroseäquivalenz einer Lösung von ≥ 4 Gew.-% bevorzugt ≥ 6 Gew.-% Sucrose in Wasser besitzt.

Überraschenderweise lässt sich bei oral konsumierbaren Produkten bei denen die Konzentration der Stoffe der Gruppe (i) so eingestellt ist, dass sie eine Sucrose-Äquivalenz von größer gleich 4 Gew.-%, bevorzugt größer gleich 6 Gew.% Sucrose in Wasser besitzt, der durch Phyllodulcin bzw. seine Salze ausgelöste synergistische Verstärkungseffekt der Süßwirkung besonders gut erzielen.

Bevorzugt sind in den erfindungsgemäßen oral konsumierbaren Produkten die Obergrenze für Phyllodulcin 15 ppm, weiter bevorzugt 10 ppm, weiter bevorzugt 5 ppm und - sofern vorhanden - die Obergrenze für Hydrangenol 50 ppm, wobei die bevorzugte Untergrenze für Hydrangenol bei 0,01 ppm liegt, jeweils bezogen auf das Gesamtgewicht des oral konsumierbaren Produktes.

Erfindungsgemäß ist es des Weiteren bevorzugt, dass sofern ein Extrakt aus *Hydrangea dulcis* im erfindungsgemäßen Produkt umfasst ist, dass dieser Extrakt auch mit einer nicht-süßen Aromanote zum Gesamtaroma des Produktes beiträgt.

Erfindungsgemäß bevorzugt ist eine erfindungsgemäße Aromamischung, bei der der oder die Stoffe der Gruppe (iii) ausgewählt sind aus Gruppe bestehend aus
- Hesperetin
- Phloretin
- 3',7-Dihydroxy-4'-methoxyflavan und
- (S)-3',7-Dihydroxy-4'-methoxyflavan.

Des Weiteren ist eine erfindungsgemäße Aromamischung bevorzugt, die einen oder weitere Aroma-, Hilfs- und/oder Trägerstoffe umfasst.

In den erfindungsgemäßen Aromamischungen ist es darüber hinaus bevorzugt, dass Phyllodulcin in der S-Form (vgl. Formel S-1) angereichert vorliegt. Bevorzugt liegt der Enatiomerenüberschuss zwischen 1 und 100 %.

Ganz besonders bevorzugt ist erfindungsgemäß ein oral konsumierbares süß schmeckendes Produkt, umfassend
(i) einen oder mehreren natürlich vorkommenden süß schmeckende Stoffe einschließlich deren physiologisch verträglichen Salze, vorzugsweise ausgewählt aus der Gruppe bestehend aus:
   a) Kohlenhydraten (Zucker), ausgewählt aus der Untergruppe bestehend aus Sucrose, D-Fructose, D-Glucose und pflanzliche Zubereitungen enthaltend einen oder mehrere der genannten Kohlenhydrate, (vorzugsweise in einem Anteil von zumindest 5 Gew. %, bevorzugt zumindest 15 Gew.-%), wobei diese Kohlenhydrate auch als natürliche oder künstlich hergestellte Mischung vorliegen können (z.B. als Honig, Invertzuckersirup, hochangereicherte Fructose-Sirupe aus Maisstärke [High Fructose Corn Sirup]);
   b) Zuckeralkoholen ausgewählt aus der Untergruppe bestehend aus Glycerin und Erythritol,
   d) Süßstoffen ausgewählt aus der Untergruppe bestehend aus Steviosid, Rebaudiosid A und Rubusosid, wobei auch Extrakte oder angereicherte Fraktionen dieser Extrakte verwendet werden können, z.B. Stevia-Extrakte und *Rubus suavissimus-*Extrakte
      alleine oder in Mischungen
      mit der Maßgabe, dass die Süße der genannten süß schmeckenden Stoffe der Gruppe (i) gemeinsam in dem erfindungsgemäßen oral konsumierbaren süß schmeckenden Produkt sensorisch äquivalent zu einer mindestens 6 %igen wässrigen Sucroselösung ist,
      und
(ii) einen Extrakt aus *Hydrangea macrophylla var.* thunbergii (Makino), umfassend Phyllodulcin und Hydrangenol in einer Konzentration, so dass bezogen auf das oral konsumierbare süß schmeckende Produkt mindestens 0,00005 Gew.-% (0,5 ppm) und höchstens 0,0015 Gew.-% (15 ppm) Phyllodulcin und mindestens 0,000001 Gew.-% und höchstens 0,0050 Gew.-% Hydrangenol enthalten sind, wobei der Extrakt auch eine nicht-süße Aroma- und Geschmackseigenschaft zeigt
   und
(iii) einen oder mehrere süßverstärkende Aroma- und/oder Geschmackstoffe einschließlich deren physiologisch verträglichen Salze, jeweils ausgewählt aus der Gruppe bestehend aus
   - Hesperetin
   - Phloretin
   - 3',7-Dihydroxy-4'-methoxyflavan oder (S)-3',7-Dihydroxy-4'-methoxyflavan
      und gegebenenfalls
(iv) einen oder mehrere weitere übliche Lebensmittelbestandteile, Aroma-, Hitfs- oder Trägerstoffe.

In den erfindungsgemäßen oral konsumierbaren, süß schmeckenden Produkten sind wie erwähnt bevorzugt die Konzentrationen der Verbindungen der Gruppe (i) insgesamt sensorisch äquivalent zu einer ≥ 4 Gew.-%igen, bevorzugt 6 Gew.-%igen wässrigen Sucroselösung. Dabei ist aber das Verhältnis der natürlich vorkommenden süß schmeckenden Stoffe aus Gruppe (i) untereinander frei wählbar. Bevorzugt werden hier die Verbindungen der Gruppen (i) a) und/oder (i) (b) in einer Konzentration sensorisch äquivalent zu einer mindestens 3 Gew.%igen, bevorzugt 5 Gew.-%igen wässrigen Sucroselösung und die Verbindungen der Gruppen (i) c) und/oder (i) d) in einer Konzentration sensorisch äquivalent zu einer höchstens 2 Gew.-%igen, bevorzugt höchstens 1 Gew.%igen wässrigen Sucroselösung eingesetzt. Auf die Weise lässt sich eine Zuckerreduzierung besonders gut erzielen, ohne dass der Geschmackseindruck beeinträchtigt wird.

Überraschenderweise wurde gefunden, dass in den erfindungsgemäßen, oral konsumierbaren süß schmeckenden Produkten die Süße wesentlich höher (nicht additiv, sondern synergistisch verstärkt, vergleiche auch oben und Beispiele), als in Vergleichsprodukten ist die nicht diese erfindungsgemäße Kombination enthalten. Vor allem die Verwendung des Extrakts in der oben beschriebenen Form und Konzentration ist vorteilhaft durch die vorhandenen Aromaeigenschaften, die das Süßprofil der erfindungsgemäßen, oral konsumierbaren süß schmeckenden Produkte abrundet und zuckerähnlicher und beständiger macht.

Bevorzugt ist ein erfindungsgemäß oral konsumierbares Produkt, ausgewählt aus der Gruppe bestehend aus pharmazeutischer Zubereitung, der Mundpflege dienende Zubereitung, Halbfertigware und flüssigem oder festem Lebensmittel.

Besonders bevorzugt ist das oral konsumierbare Produkt ein süß schmeckendes Getränk, das auch carbonisiert sein kann, umfassend
(i) einen oder mehreren natürlich vorkommenden süß schmeckende Stoffe einschließlich deren physiologisch verträglichen Salze, vorzugsweise ausgewählt aus der Gruppe bestehend aus:
   a) Kohlenhydraten (Zucker) ausgewählt aus der Untergruppe bestehend aus Sucrose, D-Fructose, D-Glucose und hochangereicherte Fructose-Sirupe aus Maisstärke [High Fructose Corn Sirup])
   d Süßstoffen ausgewählt aus der Untergruppe bestehend aus Steviosid, Rebaudiosid A und Rubusosid, wobei auch Extrakte oder angereicherte Fraktionen dieser Extrakte verwendet werden können, z.B. Stevia-Extrakte und *Rubus suavissimus-*Extrakte,
      alleine oder in Mischungen
      mit den Maßgaben, dass erstens aus Gruppen a) und d) jeweils mindestens ein süß schmeckender Stoff ausgewählt wird
      und
      dass die Süße der genannten süß schmeckenden Stoffe in der Mischung in dem erfindungsgemäßen oral konsumierbaren süß schmeckenden Produkt sensorisch äquivalent zu einer mindestens 6 %igen wässrigen Sucroselösung ist und
      bevorzugt mit der weiteren Maßgabe, dass die Summe der Konzentration der Verbindungen der Gruppen (i) a) und/oder (i) (b) sensorisch äquivalent zu einer mindestens 5 %igen wässrigen Sucroselösung und die der Gruppe (i) d) sensorisch äquivalent zu einer höchstens 1 %igen wässrigen Sucroselösung sind,
      und
(ii) einen Extrakt aus *Hydrangea macrophylla var.* thunbergii (Makino), umfassend Phyllodulcin und Hydrangenol in einer Konzentration, so dass bezogen auf das oral konsumierbare süß schmeckende Produkt mindestens 0,00005 Gew.-% (0,5 ppm) und höchstens 0,0015 Gew.-% (15 ppm) Phyllodulcin und mindestens 0,000001 % und höchstens 0,0050 % Hydrangenol enthalten sind, wobei der Extrakt auch eine nicht-süße Aroma- und Geschmackseigenschaft zeigt,
   und
(iii) einen oder mehrere süßverstärkende Aroma- und/oder Geschmackstoffe einschließlich deren physiologisch verträglichen Salze, ausgewählt aus der Gruppe bestehend aus
   - Hesperetin
   - Phloretin
   - 3',7-Dihydroxy-4'-methoxyflavan und
   - (S)-3',7-Dihydroxy-4'-methoxyflavan
      und gegebenenfalls
(iv) einen oder mehrere weitere Aroma-, Hilfs- oder Trägerstoffe.

Bevorzugte erfindungsgemäße (carbonsierte) süß schmeckende Getränke sind carbonisierte, säure- und fruchthaltige Limonaden (beispielsweise Orangen-, Limonen- oder Zitronentyp), carbonisierte isotonische Getränke (beispielsweise Orangen-, Limonen oder Zitronentyp), carbonisierte, saure Erfrischungsgetränke (beispielsweise Cola-, Zitrone-, Orange-, Limone-, Kirsch-, Apfel, Vanille-Typ oder deren Mischung), carbonisierte Schorlen, carbonisierte Obst- und Gemüsesäfte, carbonisierte Frucht- oder Gemüsesaftzubereitungen.

Dabei bedeuted carbonisiert im Sinne der Erfindung, dass das Getränk natürlich (z.B. aus Gärungsprozessen wie bei der Bierherstellung oder durch Wasser aus Kohlendioxidhaltigen Mineralquellen) eingetragenes Kohlendioxid enthält oder diesem Kohlendioxid während des Herstell- und/oder Abfüllprozesses zugesetzt wurde.

Bevorzugte Hilfs- oder Trägerstoffe sind Maltodextrin, Stärke, natürliche oder künstliche Polysaccharide und/oder Pflanzengummen wie modifizierte Stärken oder Gummi Arabicum, für die Aromamischungen zugelassene Lösungsmittel wie beispielsweise Ethanol, 1,2-Propylenglycol, Wasser, Glycerin, Triacetin, Pflanzenöltriglyceride, färbende Mittel, beispielsweise zugelassene Lebensmittetfarbstoffe, färbende Pflanzenextrakte, Stabilisitoren, Konservierungsstoffe, Antioxidantien und viskositäte-beeinflussende Stoffe.

Ein bevorzugtes erfindungsgemäß oral konsumierbares süß schmeckendes Produkt enthält die Komponenten der Gruppe
(i a) von 1 bis 90 Gew.-%, bevorzugt 5 bis 30 Gew.-%,
(i b) von 1 bis 90 Gew.-%, bevorzugt 1 bis 20 Gew.-%,
(i c) von 0,001 bis 1 Gew.-%, bevorzugt 0,01 bis 0,5 Gew.-%,
(i d) von 0,0001 bis 0,5 Gew.-%, bevorzugt 0,0001 bis 0,1 Gew.%;
(ii) mindestens 0,5 ppm und höchstens 15 ppm Phyllodulcin und mindestens 0,000001 Gew.-% und höchstens 0,0050 Gew.-% Hydrangenol;
(iii) von 0,0001 bis 0,1 Gew.%, bevorzugt 0,0005 bis 0,05 Gew.-%; und
(iv) von 0 bis 99 Gew.%, bevorzugt von 10 bis 95 Gew.-%
   jeweils bezogen auf das Gewicht des gesamten, oral konsumierbaren süß schmeckenden Produkts.

Wie bereits beschrieben umfassen bevorzugte erfindungsgemäße oral konsumierbare Produkte sowie bevorzugte erfindungsgemäße Aromamischungen weitere Aromastoffe.

Bevorzugte Aromastoffe sind Aromastoffe, die einen süßen Geruchseindruck verursachen, wobei der oder die weiteren Aromastoffe, die einen süßen Geruchseindruck verursachen, bevorzugt ausgewählt sind aus der Gruppe bestehend aus:

Vanillin, Ethylvanillin, Ethylvanillinisobutyrat (= 3-Ethoxy-4-isobutyryloxybenzaldehyd), Furaneol^{®} (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxypheny)-2-butanon, 1,1-Dimethoxy-2,2,5-trimethyl-4-hexan, 2,6-Dimethyl-5-hepten-1-al, 4-Hydroxyzimtsäure, 4-Methoxy-3-hydroxyzimtsäure, 3-Methoxy-4-hydroxyzimtsäure, 2-Hydroxyzimtsäure, 2,4-Dihydroxybenzoesäure, 3-Hydroxybenzoesäure, 3,4-Dihydroxybenzoesäure, Vanillinsäure, Homovanillinsäure, Vanillomandelsäure und Phenylacetaldehyd.

Eine besonders bevorzugte erfindungsgemäße Aromamischung oder ein erfindungsgemäß oral konsumierbares Produkt umfasst außerdem zumindest einen Aromastoff zum Verstärken eines salzigen, gegebenenfalls leicht sauren und/oder Umami-Geschmackseindrucks. Es werden somit die erfindungsgemäßen Aromamischungen und Produkte in Kombination mit zumindest einer (weiteren) zur Verstärkung eines angenehmen Geschmackseindrucks (salzig, Umami, gegebenenfalls leicht sauer) geeigneten Substanz verwendet. Hierbei bevorzugt sind salzig schmeckende Verbindungen und salzverstärkende Verbindungen. Bevorzugte Verbindungen sind in der WO 2007/045566 offenbart. Ferner bevorzugt sind Umami-Verbindungen wie in der WO 2008/046895 und EP 1 989 944 beschrieben sind.

Weiterhin können erfindungsgemäß bevorzugte Aromamischungen und Produkte auch Aromastoffe zur Maskierung von bitteren und/oder adstringierenden Geschmackseindrücken umfassen (Geschmackskorrigentien). Die (weiteren) Geschmackskorrigenzien werden z. B. aus der folgenden Liste ausgewählt: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid, 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), bittermaskierende Hydroxydeoxybenzoine z.B. gemäß WO 2006/106023 (z.B. 2-(4-Hydroxy-3-methoxyphenyl)-1-(2,4,6-trihydroxy-phenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon),), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen gemäß PCT/EP 2006/067120 Diacetyltrimere gemäß WO 2006/058893, Gemische von Molkeproteinen mit Lecithinen und/oder bittermaskierende Substanzen wie Gingerdione gemäß WO 2007/003527.

Die oral konsumierbaren süß schmeckenden Produkte im Sinne der Erfindung sind beispielsweise Backwaren (beispielsweise Brot, Trockenkekse, Kuchen, sonstiges Gebäck), Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithinhaltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tieflcühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Die oral konsumierbaren süß schmeckenden Produkte im Sinne der Erfindung können auch als Halbfertigware zur Herstellung weiterer oral konsumierbarer süß schmeckender Produkte dienen. Die oral konsumierbaren süß schmeckenden Produkte im Sinne der Erfindung können auch in Form von Kapseln, Tabletten (nichtüberzogene sowie überzogene Tabletten, beispielsweise magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Besonders bevorzugte oral konsumierbare süß schmeckende Produkte im Sinne der Erfindung sind alkoholische Getränke wie Biermixgetränke, Weinmixgetränke oder sonstige, maximal 5 Vol.-% Alkohol enthaltende Mischgetränke und/oder nichtalkoholische Getränke wie Tee, Eistee (gesüßt, beispielsweise auch mit Kräuteraromen oder Fruchtaromen vom Typ Zitrone, Orange), (carbonisierte) fruchthaltige Limonaden (beispielsweise Orangen-, Limonen- oder Zitronentyp), (carbonisierte) isotonische Getränke (beispielsweise Orangen-, Limonen oder Zitronentyp), (carbonisierte) Erfrischungsgetränke (beispielsweise Cola-, Zitrone-, Orange-, Limone-, Kirsch-, Apfel-, Vanille-Typ oder deren Mischung), Nektare, Schorlen, Milchgetränke, Buttermilchgetränke, Joghurt, Kefir, Molkegetränke, Sojamilch und daraus gefertigte Produkte, Fruchtgetränke mit Sojaprotein, Haferprotein-Getränke, und Instantgetränke (beispielsweise Instant-Kakao-Getränke, Instant-Tee-Getränke, Instant-Kaffeegetränke, Instant-Fruchtgetränke), sogenannte aromatisierte Wässer ("Near Water"-Getränke, wobei letztere eine Süßung haben müssen.

"Near Water"-Getränke im Sinne dieses Textes sind (carbonisierte) Getränke auf (Mineral-)Wasserbasis, die meist klar sind, nur schwach gefärbt werden, oft nur schwach gesüßt sind (weniger als 5 % Sucrose oder Süßstoffe mit einer Süßkraft von weniger als 5 % Sucrose), meist gar nicht oder nur schwach angesäuert sind und dabei einen pH-Bereich von ca. 4 bis 8 umfassen meist nur aromatisiert sind und dabei noch mit Mineralien, Vitaminen und/oder Pflanzenextrakten versehen werden können. Im Gegensatz zu den meisten andere Getränken (z.B. Limonaden, Fruchtsaftgetränken, [Eis-]Teegetränken u.s.w.) steht dabei der "Wasser"-Charakter des Getränks immer noch im Vordergrund.

Bevorzugt sind dabei die Getränke, die einen pH-Wert kleiner 7, besonders bevorzugt kleiner 5, insbesondere bevorzugt kleiner 4 haben.

Erfindungsgemäße oral konsumierbare süß schmeckende Produkte können auch der Mundpflege dienende oder Pharmazeutische Zubereitungen sein.

Der Mundpflege dienende Zubereitungen im Sinne dieses Textes sind insbesondere Mund- und/oder Zahnpflegemittel wie Zahnpasten, Zahngele, Zahnpulver, Mundwässer, Kaugummis und andere Mundpflegemittel.

Pharmazeutische Zubereitungen umfassen einen pharmazeutischen Wirkstoff. Vorteilhafte pharmazeutische Wirkstoffe sind beispielsweise steroidale entzündungshemmende Substanzen vom Kortikosteroiden-Typ wie beispielsweise Hydrocortison, Hydrocortison-Derivate wie Hydrocortison-17-butyrat, Dexamethason, Dexamethasonphosphat, Methylprednisolon oder Cortison.

Vorteilhafte nichtsteroidale pharmazeutische Wirkstoffe sind beispielsweise Entzündungshemmer wie Oxicame wie Piroxicam oder Tenoxicam; Salicylate wie Aspirin@ (Acetylsalicylsäure), Disalcid, Solprin oder Fendosal; Essigsäure-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Flurbiprofen, Benoxaprofen oder Pyrazole wie Phenylbutazon, Oxyphenylbutazon, Febrazon oder Azapropazon.

Besonders bevorzugte pharmazeutische Zubereitungen sind nicht verschreibungspflichtige Produkte und frei verkäufliche Arzneimittel, sogenannte OTC ("over the counter") - Präparate, enthaltend Wirkstoffe wie Paracetamol, Acetylsalicylsäure oder Ibuprofen, Vitamine (beispielsweise Vitamin H, Vitamine aus der B-Reihe wie Vitamin B1, B2, B6, B12, Niacin, Panthotensäure, vorzugsweise in Form von (Brause)Tabletten oder Kapseln), Mineralien (vorzugsweise in Form von (Brause)Tabletten oder Kapseln) wie Eisensalze, Zinksalze, Selensalze, Produkte enthaltend Wirkstoffe oder Extrakte von Spitzwegerich (beispielsweise in Hustensirup) oder Johanniskraut.

Weitere übliche Lebensmittelinhaltsstoffe können Wirk-, Grund-, Hitfs- und Zusatzstoffe sein. Weitere übliche Wirk-, Grund-, Hilfs- und Zusatzstoffe für oral konsumierbare süß schmeckende Produkte können in Mengen von 5 bis 99,999999 Gew.%, vorzugsweise 10 bis 80 Gew.-%, bezogen auf das Gesamtgewicht der Zubereitung, enthalten sein. Ferner können die oral konsumierbare süß schmeckende Produkte Wasser in einer Menge bis zu 99,999999 Gew.-%, vorzugsweise 5 bis 80 Gew.-%, bezogen auf das Gesamtgewicht der Zubereitung, aufweisen.

Bestandteil der Erfindung ist auch ein Verfahren zur Herstellung eines süßmittelreduzierten oral konsumierbaren Produktes, umfassend die Schritte:
a) Bereitstellen einer Aromamischung oder-der einzelnen Bestandteile eines solchen Aromamischung und
b) Einarbeiten der in Schritt a) bereitgestellten Aromamischung oder Bestandteile der Aromamischung in ein oral konsumierbares Produkt, so dass das Verhältnis des Sucroseäquivalenz der Konzentration des Stoffes oder der Stoffe der Gruppe (i) zur Sucroseäquivalenz der Konzentration des Stoffes oder der Stoffe der Gruppe (ii) im fertig hergestellten oral konsumierbaren Produkt ≥ 2, bevorzugt ≥ 3, weiter bevorzugt ≥ 4 und besonders bevorzugt ≥ 6.

Dabei ist ein erfindungsgemäßes Verfahren bevorzugt, wobei Schritt b) erfolgt, dass im fertig hergestellten oral konsumierbaren Produkt die Konzentration der Stoffe der Gruppe (ii) 0,5 bis 15 ppm beträgt, bezogen auf des Gewicht des Produktes und/oder die Konzentration der Stoffe der Gruppe (i) alleine eine Sucroseäquivalenz einer Lösung von ≥ 4 Gew.-% bevorzugt ≥ 6 Gew.-% Sucrose in Wasser besitzt.

Die erfindungsgemäßen Produkte werden gemäß einer bevorzugten Ausgestaltung hergestellt, indem die Bestandteile der Gruppen (i), (ii) und ggfs. auch (iii) der erfindungsgemäßen Aromamischung als Substanzen, als Lösung oder in Form eines Gemischs mit einem festen oder flüssigen Trägerstoff in eine der Ernährung, der Mundpflege oder dem Genuss dienende oder orale pharmazeutische Basis-Zubereitung eingearbeitet werden. Vorteilhafterweise können als Lösung vorliegende erfindungsgemäße Zubereitungen auch durch Sprühtrocknung in eine feste Zubereitung überführt werden.

Gemäß einer weiteren bevorzugten Ausführungsform werden zur Herstellung erfindungsgemäßer oral konsumierbarer Produkte erfindungsgemäße Aromamischungen in Form von Emulsionen, in Liposomen, beispielsweise ausgehend von Phosphatidylcholin, in Microsphären, in Nanosphären oder auch in Kapseln, Granulaten oder Extrudaten aus einer für Lebens- und Genussmittel geeigneten Matrix, beispielsweise aus Stärke, Stärkederivaten, Cellulose oder Cellulosederivaten (beispielsweise Hydroxypropylcellulose), anderen Polysacchariden (beispielsweise Alginat), natürlichen Fetten, natürlichen Wachsen (beispielsweise Bienenwachs, Carnaubawachs) oder aus Proteinen, beispielsweise Gelatine, eingearbeitet.

In einem weiteren bevorzugten Herstellungsverfahren wird die erfindungsgemäße Aromamischung mit einem oder mehreren geeigneten Komplexbildnern, beispielsweise mit Cycloglycanen, beispielsweise Cyclofructanen, Cyclodextrinen oder Cyclodextrinderivaten, bevorzugt α-, γ- und β-Cyclodextrin, komplexiert und in dieser komplexierten Form eingesetzt.

Besonders bevorzugt ist eine erfindungsgemäßes oral konsumierbares süß schmeckendes Produkt, bei der die Matrix so gewählt wird, dass die erfindungsgemäße Aromamischung verzögert von der Matrix freigegeben wird, so dass man eine langanhaltende Wirkung erhält.

Als weitere Bestandteile für erfindungsgemäße oral konsumierbare süß schmeckende Produkte können übliche Grund-, Hilfs- und Zusatzstoffe für Nahrungs- oder Genussmittel verwendet werden.

Beispiele üblicher Grund-, Hilfs- und Zusatzstoffe sind Wasser, Gemische frischer oder prozessierter, pflanzlicher oder tierischer Grund- oder Rohstoffe (beispielsweise rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (beispielsweise Dextrine, Amylose, Amylopektin, Inulin, Xylane, Cellulose), natürliche oder gehärtete Fette (beispielsweise Talg, Schmalz, Palmfett, Kokosfett, gehärtetes Pflanzenfett), Öle (beispielsweise Sonnenblumenöl, Erdnussöl, Maiskeimöl, Olivenöl, Fischöl, Sojaöl, Sesamöl), Fettsäuren oder deren Salze (beispielsweise Kaliumstearat), proteinogene oder nicht-proteinogene Aminosäuren und verwandte Verbindungen (beispielsweise Taurin), Peptide, native oder prozessierte Proteine (beispielsweise Gelatine), Enzyme (beispielsweise Peptidasen), Nukleinsäuren, Nucleotide, andere als die vorbeschriebenen Geschmackskorrigenzien für unangenehme Geschmackseindrücke, Geschmackskorrigenzien für weitere, in der Regel nicht unangenehme Geschmackseindrücke, geschmacksmodulierende Stoffe (beispielsweise Inositolphosphat, Nucleotide wie Guanosinmonophosphat, Adenosinmonophosphat oder andere Stoffe wie Natriumglutamat oder 2-Phenoxypropionsäure), Emulgatoren (beispielsweise Lecithine, Diacylglycerole), Stabilisatoren (beispielsweise Carageenan, Alginat), Konservierungsstoffe (beispielsweise Benzoesäure, Sorbinsäure), Antioxidantien (beispielsweise Tocopherol, Ascorbinsäure), Chelatoren (beispielsweise Citronensäure), organische oder anorganische Säuerungsmittel (beispielsweise Äpfelsäure, Essigsäure, Citronensäure, Weinsäure, Phosphorsäure), zusätzliche Bitterstoffe (beispielsweise Chinin, Coffein, Limonin, Amarogentin, Humolone, Lupolone, Catechine, Tannine), mineralische Salze (beispielsweise Natriumchlorid, Kaliumchlorid, Magnesiumchlorid, Natriumphosphate), die enzymatische Bräunung verhindernde Stoffe (beispielsweise Sulfit, Ascorbinsäure), etherische Öle, Pflanzenextrakte, natürliche oder synthetische Farbstoffe oder Farbpigmente (beispielsweise Carotinoide, Flavonoide, Anthocyane, Chlorophyll und deren Derivate), Gewürze, synthetische, natürliche oder naturidentische Aromastoffe oder Riechstoffe sowie Geruchskorrigenzien.

Zahnpflegemittel (als Basis für die Mundpflege dienende Zubereitungen), die die erfindungsgemäße Aromamischung enthalten, umfassen bevorzugt ein abrasives System (Schleif oder Poliermittel), wie beispielsweise Kieselsäuren, Calciumcarbonate, Calciumphosphate, Alumiuniumoxide und/oder Hydroxylapatite, oberflächenaktive Substanzen wie beispielsweise Natriumlaurylsulfat, Natriumlaurylsarcosinat und/oder Cocamidopropylbetain, Feuchthaltemitteln wie beispielsweise Glycerin und/oder Sorbit, Verdickungsmittel, wie beispielsweise Carboxymethylcellulose, Polyethylenglycole, Carrageenan und/oder Laponite^{®}, andere Geschmackskorrigenzien für unangenehme Geschmackseindrücke, Geschmackskorrigenzien für weitere, in der Regel nicht unangenehme Geschmackseindrücke, geschmacksmodulierende Stoffe (beispielsweise Inositolphosphat, Nucleotide wie Guanosinmonophosphat, Adenosinmonophosphat oder andere Stoffe wie Natriumglutamat oder 2-Phenoxypropionsäure), Kühlwirkstoffen wie beispielsweise Menthol, Mentholderivate (beispielsweise L-Menthol, L-Menthyllactat, L-Menthylalkylcarbonate, Menthonketale, Menthancarbonsäureamide), 2,2,2-Trialkylessigsäureamiden (beispielsweise 2,2-Diisopropylpropionsäureinethylamid), Icilin-Derivate, Stabilisatoren und aktive Wirkstoffe, wie beispielsweise Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen, Natriumbicarbonat und/oder Geruchskorrigenzien.

Kaugummis (als weiteres Beispiel für die Mundpflege dienende Zubereitungen), welche eine erfindungsgemäße Aromamischung enthalten, umfassen bevorzugt eine Kaugummibase, d.h. eine beim Kauen plastisch werdende Kaumasse, Zucker verschiedener Arten, Zuckeraustauschstoffe, Süßstoffe, Zuckeralkoholen, andere Geschmackskorrigenzien für unangenehme Geschmackseindrücke, Geschmackskorrigenzien für weitere, in der Regel nicht unangenehme Geschmackseindrücke, geschmacksmodulierende Stoffe (beispielsweise Inositolphosphat, Nucleotide wie Guanosinmonophosphat, Adenosinmonophosphat oder andere Stoffe wie Natriumglutamat oder 2-Phenoxypropionsäure), die im vorherigen Abschnitt genannten Kühlwirkstoffe, Feuchthaltemittel, Verdicker, Emulgatoren, Aromen, Stabilisatoren und/oder Geruchskorrigenzien.

Die erfindungsgemäßen oral konsumierbaren süß schmeckenden Produkte sind regelmäßig Produkte, die dazu bestimmt sind, in die menschliche Mundhöhle eingebracht zu werden, dort eine bestimmte Zeit zu verbleiben und anschließend entweder verzehrt (beispielsweise verzehrfertige Lebensmittel) oder wieder aus der Mundhöhle entfernt zu werden (beispielsweise Kaugummis oder Zahnpasta). Es versteht sich, dass der Einsatz der erfindungsgemäßen Aromamischung für jede Art solcher Produkte vorgesehen sein kann. Zu diesen Produkten gehören dabei sämtliche Stoffe oder Erzeugnisse, die dazu bestimmt sind, in verarbeitetem, teilweise verarbeitetem oder unverarbeitetem Zustand vom Menschen in die Mundhöhle aufgenommen zu werden. Hierzu zählen auch Stoffe, die Lebensmitteln bei ihrer Herstellung, Verarbeitung oder Bearbeitung zugesetzt werden und dazu vorgesehen sind, in die menschliche Mundhöhle eingebracht zu werden.

Es versteht sich, dass die erfindungsgemäße Aromamischung insbesondere in Lebensmitteln eingesetzt werden kann. Im Rahmen des vorliegenden Textes werden unter einem "Lebensmittel." insbesondere Stoffe verstanden, die dazu bestimmt sind, in unverändertem, zubereitetem oder verarbeitetem Zustand vom Menschen geschluckt und dann verdaut zu werden; als Lebensmittel werden insoweit auch Umhüllungen, Überzüge oder sonstige Umschließungen verstanden, die dazu bestimmt sind, mitverschluckt zu werden, oder bei denen ein Verschlucken vorauszusehen ist. Auch bestimmte Produkte, die üblicherweise wieder aus der Mundhöhle entfernt werden (beispielsweise Kaugummis) sind im Rahmen des vorliegenden Textes als Lebensmittel zu verstehen, da bei ihnen nicht auszuschließen ist, dass sie zumindest teilweise geschluckt werden.

Insbesondere wird die erfindungsgemäße Aromamischung in verzehrfertigen Lebensmitteln eingesetzt. Unter einem verzehrfertigen Lebensmittel ist hierbei ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch entsprechende Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln.

Unter einer der Mundpflege dienenden Zubereitung (Mundpflegeprodukt, auch Mundhygieneprodukt oder mundhygienische Zubereitung genannt) im Sinne der Erfindung wird eine Zubereitung zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind Cremes, Gele, Pasten, Schäume, Emulsionen, Suspensionen, Areosole, Sprays als auch Kapseln, Granulate, Pastillen, Tabletten, Bonbons oder Kaugummis, ohne dass diese Aufzählung für die Zwecke dieser Erfindung limitierend verstanden werden soll.

Die erfindungsgemäße Aromamischung kann wie bereits gesagt auch in Lebensmittel-Halbfertigwaren eingesetzt werden. Der Begriff Lebensmittel-Halbfertigware bezieht sich hierbei auf Lebensmittel, die dazu bestimmt sind, erst im weiter verarbeiteten Zustand, z. B. nach Hinzufügen von für den sensorischen Eindruck (mit)entscheidenden Aroma- oder Geschmacksstoffen verzehrt zu werden.

Gemäß dem oben Gesagten ist Bestandteil der Erfindung auch die Verwendung einer erfindungsgemäßen Aromamischung zur Herstellung eines süßmittelreduzierten oral konsumierbaren Produktes.

Wie bereits oben umfangreich beschrieben ist es durch diese Verwendung möglich, einen gegebenen Geschmackseindruck (Süße) zu erzeugen, bei gleichzeitig verringertem Einsatz von süß schmeckenden Substanzen gemäß der Gruppe (i).

Außerdem ist Bestandteil der Erfindung auch die Verwendung eines Extraktes aus *Hydrangea dulcis* zum Erzeugen eines oral konsumierbaren Produktes mit einem stabilisierten Gehalt einer Verbindungen oder an Verbindungen, ausgewählt aus der Gruppe (ii) bestehend aus Phyllodulcin und dessen physiologisch verträglichen Salzen, wobei das Produkt einen oder mehrere süß schmeckende Stoffe, ausgewählt aus der Gruppe (i) der natürlich vorkommenden süß schmeckenden Stoffe und deren physiologisch verträglichen Salzen ohne Phyllodulcin und dessen physiologisch verträglichen Salzen umfasst. Auf diese Art ist es möglich, Aromamischungen und oral konsumierbare Endprodukte bereitzustellen, in denen der Anteil an Phyllodulcin bzw. dessen Salzen stabilisiert ist, so dass der vorteilhafte Effekt dieser Verbindungen auch über einen verringerten Zeitraum genutzt werden kann.

Nachfolgend wird die Erfindung anhand von Beispielen und den Ansprüchen weiter erläutert. Die Beispiele dienen zur Verdeutlichung der Erfindung, ohne den Schutzbereich der Patentansprüche einzuschränken. Sofern nicht anders angegeben, beziehen sich alle Angaben auf das Gewicht.

### Beispiele

### Beispiel 1: Konsekutive Extraktion von Hydranqea dulcis

100 g direkt von/aus der lebenden Pflanze (*Hydrangea dulcis*) getrocknetes Pflanzenmaterial (hauptsächlich Blätter) werden mit entionisiertem Wasser angefeuchtet und 16 h bei Raumtemperatur stehen gelassen, so dass eine Fermentierung erfolgt. Das fermentierte Pflanzenmaterial wird bei 40°C wieder 3 h getrocknet. 100 g des fermentierten, getrockneten Pflanzenmaterials wird nachfolgen je 1 Stunde bei Raumtemperatur mit verschiedenen Lösungsmitteln in aufsteigender Polarität (Heptan, Methylenchlorid, Ethylacetat, Ethanol/Wasser 4:1) unter Rühren extrahiert. Das Lösungsmittel wird unter Vakuum entfernt und die getrockneten Extrakte in einer Dosierung von 500 ppm auf Zuckerlösung (5%) und Salzlösung (0,5%) verkostet und sensorisch bewertet.

| **Lösungsmittel** | **Ausbeute** | **Gehalt Phyllodulcin (bezogen auf die Trockenmasse)** | **Sensorische Bewertung** |
|---|---|---|---|
| Heptan | 0,8 g | 12,7% | Krautig, süß, Süßstoffnote |
| Methylenchlorid | 1,6 g | 15.4% | Süßstoffnote, Lakritze, bitter |
| Ethylacetat | 1,7 g | 10,5% | Bitter, süß, grün, krautig |
| Ethanol/Wasser | 20,2 g | 1,4% | Bitter, süß, krautig |

### Beispiel 2: Herstellung eines ethanolischen Hydrangea dulcis-Trockenextrakts

2 g getrocknetes fermentiertes Pflanzenmaterial (hergestellt wie in Beispiel 1 beschrieben) wurden in 50 ml Ethanol für eine Stunde unter Rückfluss extrahiert. Das Lösungsmittel wurde unter Vakuum entfernt und der getrocknete Extrakt in einer Dosierung von 110 ppm auf Zuckerlösung (5%) verkostet und sensorisch bewertet.

| **Lösungsmittel** | **Ausbeute** | **Gehalt Phyllodulcin (bezogen auf die Trockenmasse)** | **Sensorische Bewertung** |
|---|---|---|---|
| Ethanol | 0,29 g | 7,2% | Krautig, süß, Lakritz, Fülle, holzig |

Der beispielhafte Extrakt wurde auf seine Stabilität bei erhöhtem thermischen Stress im Vergleich zu reinem Phyllodulcin getestet. Eine Lösung von 100 mg/l Phyllodulcin bzw. 1,38 % des beispielhaften ethanolischen Extraktes (entspricht 100 mg Phyllodulcin) in einem wässrigen Phosphatpuffer, eingestellt auf pH 9, wurde direkt nach dem Ansetzen und dann in regelmäßigem wöchentlichen Abstand auf seinen Phyllodulcingehalt getestet. Dabei wurde ein Aliquot der jeweiligen Testlösung in einer HPLC-Anlage aufgetrennt (RP-Phase) und per UV-Detektion gegen eine Kalibrierung mit reinem Phyllodulcin quantifiziert. Die Lagerung der Proben erfolgte bei 40°C in geschlossenen Glasgefäßen, um Verdunstung zu vermeiden. In Figur 1 sind die Phyllodulcin-Gehalte der Testlösungen gegen die Zeit aufgetragen.

Wie in Figur 1 gut zu erkennen ist, wird reines Phyllodulcin schneller abgebaut als eine vergleichbare Menge Phyllodulcin in Form eines erfindungsgemäßen Extrakts. Grob geschätz, wird die Stabilität ca. verdreifacht.

### Beispiel 3: Herstellung eines Ethylacetat-Hydranqea dulcis- Trockenextrakts

2 g getrocknetes fermentiertes Pflanzenmaterial (hergestellt wie in Beispiel 1 beschrieben) wurden in 50 ml Ethylacetat für eine Stunde bei 40°C unter Rühren extrahiert. Das Lösungsmittel wurde unter Vakuum entfernt und der getrocknete Extrakt in einer Dosierung von 55 ppm auf Zuckerlösung (5%) verkostet und sensorisch bewertet.

| **Lösungsmittel** | **Ausbeute** | **Gehalt Phyllodulcin (bezogen auf die Trockenmasse)** | **Sensorische Bewertung** |
|---|---|---|---|
| Ethylacetat | 0,1 mg | 14,4% | Krautig, süß, süßstoffartig |

### Beispiel 4: Herstellung eines ethanolischlwässriqen Hydrangea dulcis- Trockenextrakts

2 g getrocknetes fermentiertes Pflanzenmaterial (hergestellt wie in Beispiel 1 beschrieben) wurden in 50 ml Ethanol/Wasser (7:3) für eine Stunde bei Raumtemperatur unter Rühren extrahiert. Das Lösungsmittel wurde unter Vakuum entfernt und der getrocknete Extrakt in einer Dosierung von 145 ppm auf Zuckerlösung (5%) verkostet und sensorisch bewertet.

| **Lösungsmittel** | **Ausbeute** | **Gehalt Phyllodulcin (bezogen auf die Trockenmasse)** | **Sensorische Bewertung** |
|---|---|---|---|
| Ethanol/Wasser (v/v 7:3) | 0,52 g | 5,3% | Süß, relativ neutral |

### Beispiel 5: Herstellung wässriq/ethanolische Ethvlacetat-Hvdrangea dulcis- Trockenextrakts

2 g getrocknetes fermentiertes Pflanzenmaterial (hergestellt wie in Beispiel 1 beschrieben) wurden in 50 ml Wasser/Ethanol (1:1) für eine Stunde bei 40°C unter Rühren extrahiert. Das Lösungsmittel wurde unter Vakuum entfernt und der getrocknete Extrakt in einer Dosierung von 240 ppm auf Zuckerlösung (5%) verkostet und sensorisch bewertet.

| **Lösungsmittel** | **Ausbeute** | **Gehalt Phyllodulcin (bezogen auf die Trockenmasse)** | **Sensorische Bewertung** |
|---|---|---|---|
| Wasser/Ethanol (v/v 1:1) | 0,52 g | 3,2 % | Süß, Honig |

### Anwendungsbeispiel 1: Süßverstärkunq mit Hydrangea dulcis-Extrakten

### Vorversuch: Eigensüße des Extrakts

Die Eigensüße des Extrakts aus Beispiel 2 (enthaltend 7,2 Gew.-% Phyllodulcin auf die Trockenmasse bezogen), der in verschiedenen Konzentrationen in reiner Form in Wasser aufgelöst wurde (0,0010, 0,0025, 0,0050, 0,0100, 0,0250, 0,0500 Gew.-% in Wasser), wurde anhand einer Vergleichsreihe verschiedener Sucrosekonzentrationen in Wasser (0, 0,25, 0,5 0,75, 1, 1,5, 2, 3, 4 und 5 Gew.% Sucrose in Wasser) bestimmt. Die Panelisten waren aufgefordert, jede einzelne Extrakt-Lösung gegen die Sucrose-Reihe zu testen und einzuordnen (Sucroseäquivalenz). Aus den über die Panelisten gemittelten Ergebnisse konnte die Eigensüße des Extrakts für die oben erwähnten Konzentrationen bestimmt werden.

| Konzentration des Extrakts aus Beispiel 2 in Wasser [Gew.-%] | Phyllodulcin-Konzentration in ppm | Bestimmung der Sucroseäquivalenz in Wasser | Sonstige sensorische Beschreibung |
|---|---|---|---|
| 0,0010 | 0,7 | 0,2 % | Teenote |
| 0,0025 | 1,75 | 0,45% | Teenote, Heu |
| 0,0050 | 3,5 | 0,64% | grüner Tee |
| 0,0100 | 7 | 0,83% | Fenchel, Anis, schwarzer Tee, leicht bitter |
| 0,0250 | 17,5 | 1,78% | Starke Teenote, grün, fischig, leicht bitter |
| 0,0500 | 35 | 2,7 | Süßholzwurzel, fischig, adstringierend, Tee, Heu, bitter |

Aus der Tabelle lässt sich durch Extrapolation erkennen, dass eine Sucroseäquivalenz von 1,5 % bei ca. 200 ppm des Extrakts (entsprechend ca. 15 ppm Phyllodulcin) und 1 % bei ca. 150 ppm des Extrakts (entsprechend ca. 10 ppm Phyllodulcin) erreicht werden kann.

### Verstärkung des Süßeindrucks einer Sucroselösunq

Um die Verstärkung des Süß-Eindrucks zu quantifizieren, wurde die Süße einer 5 % enthaltenden Sucroselösung und einer Probe, die 5 % Sucrose und eine Menge der Testsubstanz bzw. des Testextrakts enthielt, von einer Expertengruppe bestimmt (Einstufung 1 [nicht süß] bis 10 [extrem süß]). Die Auswertung erfolgte als Berechnung der Reduktion (in %) des Süßeindrucks aus den Durchschnittswerten der Einschätzungen der Sucroselösung bzw. der Sucrose und der Testsubstanz bzw. des Testextrakts enthaltenden Lösung.

| **Substanz/Extrakt** | **Konzentration Extrakt (ppm)/ Konzentration Phyllodulcin (ppm)** | **Süß-Eindruck (1-10)** | | **% Verstärkung des Süß-Eindrucks** |
|---|---|---|---|---|
| | | **ohne** | **mit** | |
| Ethanolischer Extrakt aus Beispiel 2 (enthält 7,2 % Phyllodulcin) | 10/0,7 | 5,0 ± 0,8 | 5,4 ± 1,5 | 8% |
| dito | 25/1,75 | 5,6± 1,2 | 5,9 ± 1,1 | 6% |
| dito | 50/3,5 | 5,3 ± 1,3 | 6,9 ± 1,5 | 31% (p < 0,01) |
| dito | 100/7 | 5,5 ± 0,9 | 7,4 ± 1,3 | 34% (p < 0,001) |
| dito | 110/7,7 | 5,6 ± 1,7 | 7,5 ± 1,8 | 33% (p < 0,01) |
| dito | 200/14 | 5,1 ± 0,8 | 7,6± 1,3 | 49 % (p < 0,001) |
| Ethylacetat-Extrakt aus Beispiel 3 (enthält 14,4 % Phyllodulcin) | 55/7,7 | 4,9 ± 1,1 | 6,9 ± 1,3 | 42% (p < 0,001) |
| Ethanolisch/wässriger Extrakt aus Beispiel 4 (enthält 5,3 % Phyllodulcin) | 145/7,7 | 5,3 ± 1,6 | 6,9 ± 1,5 | 30% (p<0,01) |
| Ethanolisch/wässriger Extrakt aus Beispiel 5 (enthält 3,2 % Phyllodulcin) | 240/7.7 | 5,3 ± 1,1 | 7,6 ± 1,6 | 43% (p<0,001) |

Man kann eine sehr gute Süßverstärkung schon bei der an sich nicht süßen Konzentration von ca. 100 ppm ethanolischem Extrakt aus Beispiel 2, entsprechend unter 10 ppm Phyllodulcin erkennen. Rein rechnerisch würde man erwarten, dass eine 5 % Sucroselösung (entspricht sensorisch definitionsgemäß 5 % Sucroseäquivalenten), die 100 ppm ethanolischem Extrakt aus Beispiel 2 enthält, additiv eine Süße von ca. 5,8 Sucroseäquivalenten ergäbe. Die sensorische Bewertung einer solchen Mischlösung aus 5 % Sucrose mit 100 ppm des erfindungsgemäßen Extrakts aus Beispiel 2 ergab nach dem im Vorversuch beschriebenen Verfahren aber eine Sucroseäquivalenz von 7,7 %, das entspricht einem Synergismus von mehr als 30 % über dem erwarteten Wert.

### Anwendungsbeispiel 2: Zuckerreduziertes Erfrischunqsqetränk

Zubereitung A: Vergleichszubereitung mit 10 % Zucker
Zubereitung B: Vergleichszubereitung mit 8 % Zucker
Zubereitung C: Vergleichszubereitung mit 8 % Zucker und Neohesperidindihydrochalkon
Zubereitung D-H: erfindungsgemäße zuckerreduzierte Zubereitungen mit 8 % Zucker

| **Inhaltsstoff** | **Einsatz in Gew.%** | | | | | | | |
|---|---|---|---|---|---|---|---|---|
| **Zubereitung** | **A** | **B** | **C** | **D** | **E** | **F** | **G** | **H** |
| Zucker | 10 | 8 | 8 | 8 | 8 | 8 | 8 | 8 |
| Zitronensäure | 0,15 | 0,15 | 0,15 | 0,15 | 0,15 | 0,15 | 0,15 | 0,15 |
| Zitronenaroma | 0,1 | 0,1 | 0,1 | 0,1 | 0,1 | 0,1 | 0,1 | 0,1 |
| Neohesperidin-dihydrochalcon | - | - | 0,0001 | - | - | - | - | - |
| Extrakt aus Beispiel - 2, enthaltend 7 % Phyllodulcin | | - | - | 0,011 | 0,005 | 0,005 | 0,005 | 0,005 |
| Phloretin | - | - | - | - | | - | 0,002 | - |
| Hesperetin | - | - | - | - | | - | - | 0,001 |
| Extrakt aus Rubus suavissimus, enthaltend 5 Gew.- % Rubusosid bez. auf das Gesamtgewicht des Extraktes | | | | | | 0,010 | | |
| Wasser | auf 100 auffüllen | | | | | | | |

Die Zutaten wurden in der angegebenen Reihenfolge gemischt und mit Wasser auf 100 % aufgefüllt. Die Mischungen werden in Glasflaschen gefüllt und carbonisiert.

Die Zubereitungen wurden sensorisch in verblindeten Duo-Tests geprüft. Dabei wurde die Süße anhand einer Einstufung 1 [nicht süß] bis 10 [extrem süß] von Experten eingeschätzt.

| **Vergleich zwischen 1. und 2. Probe** | **Süß-Eindruck (1-10)** | | **Veränderung (%)** | **Signifikanz (n.s. = nicht signifikant)** |
|---|---|---|---|---|
| | **1. Probe** | **2. Probe** | | |
| **A und B** | 7,1 ± 1,4 | 4,6 ± 1,3 | -36% | p < 0,001 |
| **A und C** | 6,3 ± 1,2 | 4,6 ± 1,9 | -28% | p < 0,01 |
| **A und D** | 6,9 ± 1,4 | 7,0 ± 1,5 | 2% | n.s. |
| **A und E** | 6,8 ± 1,8 | 5,9 ± 1,9 | -13% | n.s. |
| **A und F** | 5,4 ± 1,3 | 5,9 ± 1,4 | 9% | n.s. |
| **A und G** | 6,8 ± 1,5 | 6,3 ± 1,8 | -7% | n.s. |
| **A und H** | 6,8 ± 1,9 | 6,4 ± 2,2 | -6 % | n.s. |

Durch Weglassen von Zucker (20 Gew.-% bez. auf Sucrose) wurde eine Abnahme der Süße von ca. 36 % beobachtet (Zubereitungen A und B).

Als Vergleich (Zubereitung C) wurde der bekannte Süßstoff Neohesperidindihydrochalkon in einer Konzentration eingesetzt, die alleine in Wasser etwa 0,5 % Sucroseäquivalenz entspricht (bestimmt nach der in Anwendungsbeispiel 1, erster Teil genannten Methode). Im Vergleich zum Vergleichsbeispiel A konnte die Süße der um 20 % Sucrosereduzierten Vergleichszubereitung C allein durch 1 ppm Neohesperidindihydrochalkon nicht wiederhergestellt werden.

Durch Zugabe der erfindungsgemäßen Extrakte alleine (Zubereitungen D und E) zu der zuckerreduzierten Zubereitung konnte von den Panelisten kein *signifikanter* Unterschied mehr zwischen der Vollzucker-Zubereitung und der an Zucker ärmeren, erfindungsgemäßen Zubereitung (A gegen D bzw. A gegen E) festgestellt werden; bei der niedrigeren Dosierung (Zubereitung E) war aber noch eine nicht vollständig wieder hergestellte Süße wahrnehmbar.

Das bedeutet, dass die erfindungsgemäßen Extrakte besser als herkömmliche Süßstoffe in niedrigen Dosierungen unterhalb 1 % Sucroseäquivalent in der Lage sind, die Süße Sucrose-reduzierter Anwendungen wieder herzustellen.

Durch Kombination der geringeren Dosierung von 50 ppm des erfindungsgemäßen Extrakts nach Beispiel 2 in Kombination mit bekannten Aromastoffen zur Süßverstärkung (G, H) konnte dann aber die Süße weitgehend wieder hergestellt bzw. durch Einsatz von Extrakten aus Rubus suavissimus (F) sogar mehr Süße erzielt werden.

### Anwendungsbeispiel 3: Aromamischungen, enthaltend Süßstoffe

| | Zubereitung (Einsatz in Gew.-%) | | | | | | | | |
|---|---|---|---|---|---|---|---|---|---|
| Inhaltsstoff | A | B | C | D | E | F | G | H | I |
| "Flüssigzucker", enthält 80% Sucrose | 99,87 | - | - | - | - | - | - | - | - |
| Rebaudiosid A 98 % | - | | 80 - | - | 70 | 70 | 60 | 70 | 73,5 |
| Rebaudiosid A 90 % | - | - | 90 | - | - | - | - | - | - |
| Steviosid 95 % | - | - | - | 75 | - | - | - | - | - |
| Extrakt aus *Hydrangea* du/cis nach Beispiel 2 (ethanolisch, 7,7 % Phyllodulcin) | 0,10 | 20 | - | 20 | 20 | 10 | 10 | 20 | 20 |
| Extrakt aus *Hydrangea* du/cis nach Beispiel 3 (Ethylacetat, 14,4 % Phyllodulcin) | - | - | 10 | - | - | - | - | | - |
| Extrakt aus Rubus suavissimus, enthaltend 5 Gew.-% Rubusosid, z.B. von PlantExtrakt | - | - | - | - | - | - | 25 | - | - |
| Phloretin | 0,02 | - | - | 4 | 5 | 3,2 | 3,5 | 5 | 5 |
| Hesperetin Neohesperidin- | 0,01 | - | - | 1 | 5 | 0,8 | 1 | 4,9 | 1 |
| dihydrochalkon | - | - | - | - | - | - | 0,5 | - | - |
| Homoeriodictyol-Natriumsalz | - | - | - | - | - | 16 | - | - | - |
| Vanillin, natürlich | - | - | - | - | - | - | - | 0,1 | - |
| Zuckerdestillat aus Rohrzucker (z.B. Treatt) | - | - | - | - | - | - | - | - | 0,5 |

Die Stoffe bzw. Lösungen werden in den oben angegebenen Mengenverhältnissen gemischt und werden so verwendet. Die typische Dosierung von Zubereitung A im Fertigprodukt liegt bei 7 bis 15 Gew.-% bezogen auf das Fertigprodukt, die typische Dosierung von Zubereitungen B bis I bei 0,01 bis 0,1 % bezogen auf das Fertigprodukt, bevorzugt bei 0,03 bis 0,06 %.

### Anwendungsbeispiel 4: Sprühqetrocknete Zubereitung als Halbfertiqware zur Aromatisierunq von Fertiqwaren

| **Inhaltsstoff** | **Einsatz in Gew.%** | | | | | | | | |
|---|---|---|---|---|---|---|---|---|---|
| **Zubereitung** | **A** | **B** | **C** | **D** | **E** | **F** | **G** | **H** | **I** |
| Trinkwasser | 60 | 60 | 60 | 60 | 60 | 60 | 60 | 60 | 60 |
| Maltodextrin aus Weizen | 10 | 25 | 25 | 25 | 25 | 25 | 25 | 25 | 25 |
| Gummi Arabicum | 5 | 5 | 5 | 5 | 5 | 10 | 10 | 10 | 10 |
| Aromamischung A aus Anwendungsbeispiel 3 | 25 | - | - | - | - | - | - | - | - |
| Aromamischung B aus Anwendungsbeispiel 3 | - | 10 | - | - | - | - | - | - | - |
| Aromamischung C aus Anwendungsbeispiel 3 | - | - | 10 | - | - | - | - | - | - |
| Aromamischung D aus Anwendungsbeispiel 3 | - | - | - | 10 | - | - | - | - | - |
| Aromamischung E aus Anwendungsbeispiel 3 | - | - | - | - | 10 | - | - | - | - |
| Aromamischung F aus Anwendungsbeispiel 3 | - | - | - | - | - | 5 | - | - | - |
| Aromamischung G aus Anwendungsbeispiel 3 | - | - | - | - | - | - | 5 | - | - |
| Aromamischung H aus Anwendungsbeispiel 3 | - | - | - | - | - | - | - | 5 | - |
| Aromamischung 1 aus Anwendungsbeispiel 3 | - | - | - | - | - | - | - | - | 5 |

Das Trinkwasser wird in einem Behälter vorgelegt und das Maltodextrin und das Gummi Arabicum darin gelöst. Anschließend werden die Aromamischungen mit einem Turrax in die Trägerstofflösung emulgiert. Die Temperatur der Sprühlösung sollte 30°C nicht überschreiten. Das Gemisch wird dann sprühgetrocknet (Solltemperatur Eingang: 185 - 195°C, Solltemperatur Ausgang: 70 - 75°C).

### Anwendungsbeispiel 5: Lösungen der Aromamischungen

Die Kompositionen aus Anwendungsbeispiel 3 können auch mit Wasser, Propylenglycol, Glycerin, oder Ethanol oder bevorzugt mit Gemischen der vorgenannten Lösungsmittel (z.B. Wasser-Propylenglycol, Wasser-Glycerin, Wasser-Ethanol, Glycerin-Ethanol, Glycerin-Propylenglycol, Propylenglycol-Ethanol) beispielsweise als 1 - 20%ige Lösung, bevorzugt 2 - 10 %ige, besonders bevorzugt 5 %ige Lösung aufgenommen und durch leichtes Erwärmen vollständig gelöst werden.

### Anwendungsbeispiel 6: Softdrink Typ "Cola"

| | Zubereitung (Einsatz in Gew.-%) | | | | | | | |
|---|---|---|---|---|---|---|---|---|
| Inhaltsstoff | **A** | **B** | **C** | **D** | **E** | **F** | **G** | **H** |
| Saccharose | 0 | 8 | 7 | 7 | 7 | - | 7 | - |
| Glucose/Fructose-Sirup aus Mais, enthaltend 55 Gew.% Fructose | - | - | - | - | - | 8 | - | 7 |
| Aromamischung A aus Anwendungsbeispiel3 3 | 10 | - | - | - | - | - | - | - |
| Aromamischung B aus Anwendungsbeispiel 3 | - | 0,05 | - | - | - | - | - | - |
| Aromamischung C aus Anwendungsbeispiel 3 | - | - | 005 | - | - | - | - | - |
| Aromamischung D aus Anwendungsbeispiel 3 | - | - | - | 0,05 | - | - | - | - |
| Aromamischung E aus Anwendungsbeispiel 3 | - | - | - | - | 0,05 | - | - | - |
| Aromamischung F aus Anwendungsbeispiel 3 | - | - | - | - | - | 0,05 | - | - |
| Aromamischung G aus Anwendungsbeispiel 3 | - | - | - | - | - | - | 0,05 | - |
| Aromamischung I aus Anwendungsbeispiel 3 | - | - | - | - | - | - | - | 0,05 |
| Phosphorsäure | 0,07 | 0,07 | 0,07 | 0,07 | 0,07 | 0,07 | 0,07 | 0,07 |
| Citronensäure | 0,06 | 0,06 | 0,06 | 0,06 | 0,06 | 0,06 | 0,06 | 0,06 |
| Zuckercouleur | 0,14 | 0,14 | 0,14 | 0,14 | 0,14 | 0,14 | 0,14 | 0,14 |
| Coffein | 0,01 | 0,01 | 0,01 | 0,01 | 0,01 | 0,01 | 0,01 | 0,01 |
| Getränke-Emulsion Typ "Cola" | 0,05 | 0,05 | 0,05 | 0,05 | 0,05 | 0,05 | 0,05 | 0,05 |
| Wasser | auf 100% auffüllen | | | | | | | |

Die Zutaten wurden in der angegebenen Reihenfolge gemischt, in Flaschen gefüllt und carbonisiert.

### Anwendungsbeispiel 7: Eisteeqetränke

| | Zubereitung (Einsatz in Gew.-%) | | | | | | | |
|---|---|---|---|---|---|---|---|---|
| Inhaltsstoff | **A** | **B** | **C** | **D** | **E** | **F** | **G** | **H** |
| Aromamischung A aus Anwendungsbeispiel 3 | 6 | - | - | - | - | - | - | - |
| Aromamischung F aus Anwendungsbeispiel 3 | - | - | - | 0,03 | - | - | 0,03 | 0,03 |
| Aromamischung G aus Anwendungsbeispiel 3 | - | 0,03 | - | - | 0,03 | - | - | - |
| Aromamischung H aus Anwendungsbeispiel 3 | - | - | 0,03 | - | - | 0,03 | - | - |
| Zitronensäure | 0,15 | 0,12 | 0,15 | 0,12 | 0,15 | 0,12 | 0,15 | 0,12 |
| Ascorbinsäure | 0,02 | 0,02 | 0,02 | 0,02 | 0,02 | 0,02 | 0,02 | 0,02 |
| Schwarzteeextrakt | 0,15 | 0,15 | - | - | 0,15 | 0,15 | - | - |
| Grünteeextrakt (mind. 50 % Polyphenole) | - | - | 0,1 | 0,1 | - | - | 0,33 | 0,33 |
| natürliches Aroma Typ "Zitrone" | 0,1 | - | 0,1 | - | 0,1 | - | 0,1 | - |
| natürliches Aroma Typ "Pfirsich" | - | 0,07 | - | 0,07 | - | 0,07 | - | 0,07 |
| Wasser | auf 100% auffüllen | | | | | | | |

Die Zutaten wurden in der angegebenen Reihenfolge gemischt, in Flaschen gefüllt und sterilisiert.

### Anwendungsbeispiel 8: Verwendung in einem Kauqummi

| **Teil** | **Inhaltsstoff** | **Einsatz in Gew.%** |
|---|---|---|
| A | Kaugummibase, Company "Jagum T" | 30,00 |
| B | Sorbit, pulverisiert | 38,975 |
| | lsomalt^{®} (Palatinit GmbH) | 9,50 |
| | Xylit | 2,00 |
| | Mannit | 3,00 |
| | Rebaudiosid A 98 % | 0,20 |
| | Ethanolischer Extrakt aus Beispiel 2 | 0,02 |
| | Hesperetin | 0,005 |
| | Emulgum^{®} (Colloides Naturels, Inc.) | 0,30 |
| C | Sorbitol, 70% | 14,00 |
| | Glycerin | 1,00 |
| D | Mint-Aroma | 1 |

Teile A bis D werden gemischt und intensiv geknetet. Die Rohmasse kann z.B. in Form von dünnen Streifen zu verzehrsfertigen Kaugummis verarbeitet werden.

### Anwendungsbeispiel 9: Zuckerfreie Hartkaramellen

| **Inhaltsstoff** | **Gehalt (%)** | | | |
|---|---|---|---|---|
| | **A** | **B** | **C** | **D** |
| **Palatinit, Typ M** | 75,00 | 74,00 | 75,50 | 75,00 |
| Zitronensäure | - | 1,0 | 0,5 | - |
| Wasser | 24,88 | 24,842 | 23,88 | 24,844 |
| **Farbstoff gelb** | - | 0,01 | - | - |
| **Farbstoff rot** | - | - | 0,01 | - |
| **Farbstoff blau** | 0,01 - | | - | 0,01 |
| Pfefferminzaroma | 0,1 | - | - | 0,1 |
| Zitronenaroma | - | 0,1 | - | - |
| Rotfruchtaroma | - | - | 0,1 | - |
| Rebaudiosid A 98 % | - | 0,040 | - | 0,040 |
| Extrakt aus Beispiel 2 | 0,010 | 0,005 | 0,010 | 0,005 |
| Hesperetin | - | 0,001 | - | 0,001 |
| Phloretin | - | 0,002 | - | - |

Palatinit wurde ggfs. nach Zugabe der Zitronensäure mit Wasser gemischt und die Mischung bei 165 °C aufgeschmolzen und anschließend auf 115°C abgekühlt. Das Aroma und 3 die anderen Bestandteile wurden zugegeben und nach dem Durchmischen in Formen gegossen, nach dem Erstarren aus den Formen entfernt und anschließend einzeln verpackt.

### Anwendungsbeispiel 10: Zuckerreduzierter Kochpuddinq

### Zubereitung A, B: Vergleichszubereitungen mit vollem (A) bzw. reduziertem (B) Zuckergehalt

| | **Zubereitung (Angaben als Gew.-%)** | | | | | |
|---|---|---|---|---|---|---|
| **Inhaltsstoff** | **A** | **B** | **C** | **D** | **E** | **F** |
| **Sucrose** | 7,8% | 5,4% | 5,4 % | 5,4% | 5,4% | 5,4% |
| Stärke | 3,0 % | 3,0 % | 3,0 % | 3,0 % | 3,0 % | 3,0 % |
| Magermilchpulver | 1,5% | 1,5% | 1,5% | 1,5% | 1,5% | 1,5% |
| Aubygel MR50 | 0,5% | 0,5% | 0,5% | 0,5% | 0,5% | 0,5% |
| Vanilleschoten-Extrakt, sprühgetrocknet, Symrise | 0,1 % | 0,1 % | 0,1 % | 0,1 % | 0,1 % | 0,1 % |
| Extrakt aus Beispiel 2 Beispiel 2 | - | - | 0,01% | 0,005% | 0,005% | 0,005% |
| Extrakt aus Rubus suavissimus, enthaltend 5 Gew.-% Rubusosid, z.B. von PlantExtrakt | - | - | - | - | 0,010 % | 0,005 % |
| Hesperetin | - | - | - | 0,001 % | - | 0,001 % |
| Phloretin | - | - | - | 0,002 % | - | 0,002 % |
| Milch 1,5 % Fettanteil | auffüllen auf 100 % auffüllen auf 100 % | | | | | |

Die festen Stoffe wurden vorgelegt und mit der Milch aufgerührt. Die Mischung wurde auf 95°C für 2 min unter gutem Rühren aufgewärmt, abgefüllt und auf 5 - 8 °C abgekühlt.

### Anwendungsbeispiel 11: Fettarme Joghurts

### Vergleichszubereitung mit Zucker (A)

Erfindungsgemäße Zubereitungen mit Süßstoffmischung und Extrakt aus Beispiel 2) (B-D)

| | **Zubereitung (Angaben als Gew.-%)** | | | |
|---|---|---|---|---|
| **Inhaltsstoff** | **A** | **B** | **C** | **D** |
| **Sucrose** | 10 | 8 | 6 | - |
| Rebaudiosid A 98 % | - | - | - | 0,050 |
| Extrakt aus Beispiel 2 | - | 0,010 | 0,005 | 0,010 |
| Extrakt aus Rubus suavissimus, enthaltend 5 Gew.-% Rubusosid, z.B. von Plan-tExtrakt | - | - | 0,010 | - |
| Hesperetin | - | 0,001 | 0,001 | 0,001 |
| Phloretin | - | - | 0,002 | - |
| Hopmoeriodictyol-Natriumsalz | - | - | - | 0,005 |
| Joghurt, 0,1 % Fett | zu 100 % auffüllen | | | |

Die Inhaltsstoffe wurden gemischt und bei 5°C gekühlt.

### Anwendungsbeispiel 12: Milchmischqetränke

Vergleichszubereitungen mit Zucker (A)
Erfindungsgemäße Zubereitungen (B-D)

| | **Zubereitung (Angaben als Gew.-%)** | | | |
|---|---|---|---|---|
| **Inhaltsstoff** | **A** | **B** | **C** | **D** |
| **Sucrose** | 10 | 8 | 7 | - |
| Fructose | - | - | 0,5 | - |
| Rebaudiosid A 98 % | - | - | - | 0,040 |
| Extrakt aus Beispiel 2 | - | 0,010 | 0,005 | 0,010 |
| Extrakt aus Rubus suavissimus, enthaltend 5 Gew.-% Rubusosid, z.B. von Plan-tExtrakt | - | - | 0,010 | - |
| Hesperetin | - | 0,003 | 0,002 | 0,005 |
| Phloretin | - | - | 0,002 | - |
| Hopmoeriodictyol-Natriumsalz | - | - | - | 0,002 |
| H-Milch, 1,5 % Fett | zu 100 % auffüllen | | | |

Die Inhaltsstoffe wurden gemischt, mit Milch aufgefüllt, gut gerührt, in Flaschen abgefüllt und bei 5°C gekühlt gelagert.

### Anwendungsbeispiel 13: Zuckerreduziertes Tomatenketchup

Vergleichszubereitung mit Zucker (A)
Vergleichszubereitung mit reduziertem Zuckeranteil (B)
Erfindungsgemäße Zubereitungen (C-1)

| | **Zubereitung (Angaben als Gew.-%)** | | | | | | | |
|---|---|---|---|---|---|---|---|---|
| **Inhaltsstoff** | **A** | **B** | **C** | **E** | **F** | **G** | **H** | **I** |
| Kochsalz | 2 | 2 | 2 | 2 | 2 | 2 | 2 | 2 |
| Stärke, Farinex WM 55 | 1 | 1 | 1 | 1 | 1 | 1 | 1 | 1 |
| Sucrose | 12 | 9,6 | 9,2 | 8,4 | 9,6 | 9,6 | 8,4 | 4,2 |
| Tomaten-Konzentrat 2-fach | 40 | 40 | 40 | 40 | 30 | 30 | 30 | 30 |
| Glucosesirup 80 Brix | 18 | 18 | 18 | 18 | 18 | 18 | 18 | 18 |
| Branntweinessig 10% | 7 | 7 | 7 | 7 | 3 | 3 | 3 | 3 |
| Rebaudiosid A 98 % | - | - | - | - | - | - | - | 0,05 |
| Extrakt aus Beispiel2 | - | - | 0,01 | 0,005 | 0,005 | 0,01 | 0,005 | 0,01 |
| Extrakt aus Rubus suavissimus, enthaltend 5 Gew.-% Rubusosid, z.B. von PlantExtrakt | - | - | - | - | - | - | 0,01 | - |
| Hesperetin 2,5%ig in 1,2-Propylenglycol | - | - | - | - | 0,1 | - | 0,1 | - |
| Phloretin 2,5% in 1,2-Propylenglycol | - | - | - | 0,2 | 0,2 | - | - | 0,3 |
| Wasser | zu 100 % auffüllen | | | | | | | |

Die Inhaltsstoffe werden in der angegebenen Reihenfolge gemischt und das fertige Ketchup mit Hilfe eines Rührwerkes homogenisiert, in Flaschen abgefüllt und sterilisiert.

### Anwendungsbeispiel 14: Zuckerreduzierte Eiscremes

Vergleichszubereitung mit Zucker (A)
Vergleichszubereitung mit reduziertem Zuckeranteil (B)
Erfindungsgemäße Zubereitungen (C-F)

| | **Zubereitung (Gehalt in Gew.-%)** | | | | | |
|---|---|---|---|---|---|---|
| **Inhaltsstoff** | **A** | **B** | **C** | **D** | **E** | **F** |
| Pflanzenfett, Schmelzbereich 35 - 40 °C | 20,00 | 20,00 | 20,00 | 20,00 | 20,00 | 20,00 |
| Zucker (Saccharose) | 12,00 | 8,00 | 8,00 | 8,00 | 8,00 | 8,00 |
| Magermilchpulver | 5,00 | 5,00 | 5,00 | 5,00 | 5,00 | 5,00 |
| Glucosesirup 72 % Trockensubstanz | 5,00 | 5,00 | 5,00 | 5,00 | 5,00 | 5,00 |
| Emulgator SE 30 (Grindstedt Products, Dänemark) | 0,65 | 0,65 | 0,65 | 0,65 | 0,65 | 0,65 |
| Aroma, enthaltend 0,1 % Diacetyl und 1 % Vanillin | 0,20 | 0,20 | 0,20 | 0,20 | 0,20 | 0,20 |
| Extrakt aus Beispiel 2 | - | - | 0,01 | 0,005 | 0,01 | 0,005 |
| Extrakt aus Rubus suavissimus, enthaltend 5 Gew.-% Rubusosid, z.B. von PlantExtrakt | - | - | - | - | - | 0,010 |
| Hesperetin 2,5%ig in 1,2-Propylenglycol | - | - | - | 0,10 | - | 0,10 |
| Phloretin 2,5% in 1,2-Propylenglycol | - | - | - | - | 0,05 | 0,05 |
| Magermilch | zu 100 % auffüllen | | | | | |

Das Pflanzenfett wurde auf 58°C erwärmt. Magermilch und Glucosesirup wurden auf 55° erhitzt und Zucker, Magermilchpulver sowie Emulgator und Aroma hinzugefügt und die Mischung ins Pflanzenfett gegeben. Die Mischung wurde mit Hilfe eines Durchflusshochdruckhomogenisators homogenisieret (180 / 50 bar). Die erhaltene Masse wurde 1 min lang bei 78°C temperiert, anschließend auf 2 - 4 °C abgekühlt und 10 h zur Reifung bei dieser Temperatur inkubiert. Danach wurde die gereifte Masse abgefüllt und bei -18°C eingefroren gelagert.

### Anwendungsbeispiel 15: Für Diabetiker qeeiqnetes Eis

Es wurde ein für Diabetiker geeignetes Eis aus folgenden Zutaten hergestellt und in Portionen zu je 95 mL in Becher abgefüllt:

Eingedickte entrahmte Milch, Fructosesirup, Erdbeerstücke und Erbeerpüree (15%), Pflanzenfett, Diätschokoladensplitter (3,5%, mit Emulgator Sojalecithin), Molkenerzeugnis, Rote Bete Saft, Johannisbrotkernmehl, Guarkernmehl, Carrageen, Emulgator (E 471), Gelatine, Säuerungsmittel Citronensäure, 0,1 % Erdbeer-Aroma (enthaltend 1 Gew.% des Extrakts aus Beispiel 2 und und 1 Gew.% Phloretin) bezogen auf das Gesamtgewicht des Erdbeer-Aromas), Farbstoff Carotin.

Nährwert (pro 95 mL):

Eiweiss 1,8 g, Kohlenhydrate 13,3 g (davon Fructose 9,5 g), Fett 4,2 g.

### Anwendungsbeispiel 16: Diätschokolade auf Basis von Maltit

Es wurde eine für Diabetiker geeignete Schokolade aus folgenden Zutaten hergestellt und in rechteckige Tafeln gegossen:

Maltit, Haselnussmasse, Kakaobutter, Magermilchpulver, Kakaomasse, Inulin, Butterreinfett, Emulgator Sojalecithine, 0,1 % Vanille-Aroma (enthaltend Vanilleschoten-Extrakt, Vanillin und 1 Gew.-% Extrakt aus Beispiel 2 und 0,3 Gew.-% Hesperetin, bezogen auf das Gesamtgewicht des Vanille-Aromas).

Nährwert (pro 100 g):

Eiweiss 8 g, Kohlenhydrate 43 g (davon Maltit 34 g), Fett 34 g.

### Anwendungsbeispiel 17: Diätschokolade auf Basis von Fructose

Es wurde eine für Diabetiker geeignete Schokolade aus folgenden Zutaten hergestellt und in rechteckige Tafeln gegossen:

Kakaomasse, Fructose, Magermilchpulver, Kakaobutter, Inulin, Butterreinfett, Emulgator Sojalecithin, Walnüsse, Speisesalz, 0,1 % Vanille-Aroma (enthaltend Vanillin und 1 Gew.-% Extrakt aus Beispiel 2 und 0,2 Gew.-% Hesperetin und 1 Gew.-% Homoeriodictyol-Natriumsalz, bezogen auf das Gesamtgewicht des Vanille-Aromas).

Nährwert (pro 100 g):

Eiweiss 8,8 g, Kohlenhydrate 34 g (davon Fructose 23 g, Lactose 7,5 g, Saccharose 1,4 g), Fett 36 g; Ballaststoffe 18,5 (davon 12,2 g Inulin); Natrium: 0,10 g. Kakao-Anteil mindestens 50 Gew.-%.

### Anwendungsbeispiel 18: Zuckerreduzierte Müslimischung

| | **Zubereitung (Gehalt in Gew.-%)** | |
|---|---|---|
| **Inhaltsstoff** | **A** | **B** |
| Haferflocken | 17,00 | 18,90 |
| Knusprige Haferflocken Cluster | 10,00 | 12,00 |
| Reis Crispies | 16,90 | 17,80 |
| Cornflakes | 16,50 | 17,50 |
| Korinthen | 3,50 | 3,50 |
| Haselnüsse, zerhackt | 2,50 | 2,50 |
| Glucose Sirup aus Weizen, DE 30 | 9,50 | 9,50 |
| Saccharose | 20,00 | 14,00 |
| Wasser | 4,00 | 4,00 |
| Citronensäurepulver, wasserfrei | 0,10 | 0,10 |
| Aroma, enthaltend 1 Gew.-% Extrakt aus Beispiel 2 und 0,1 Gew.-% Hesperetin bezogen auf das Aroma | - | 0,20 |

Jeweils Bestandteile Nr. 1 bis 6 in einer Drehtrommel mischen (Mix 1). Jeweils Bestandteile Nr. 7 bis 9 erwärmen und Bestandteil Nr. 10 zugeben (sowie in Rezeptur B zusätzlich den Bestandteil Nr. 11 zugeben) (Mix 2). Jeweils Mix 2 zu Mix 1 geben und gut mischen. Zuletzt die resultierende Müslimischung auf ein Backblech geben und in einem Ofen bei 130°C für 8 Minuten trocknen.

### Anwendungsbeispiel 19: Zuckerreduzierte Fruchtgummis

| | **Zubereitung (Gehalt in Gew.-%)** | |
|---|---|---|
| **Inhaltsstoff** | **A** | **B** |
| Wasser | 23,70 | 25,70 |
| Saccharose | 34,50 | 8,20 |
| Glucosesirup, DE 40 | 31,89 | 30,09 |
| Iso Syrup C* Tru Sweet 01750 (Cerestar GmbH) | 1,50 | 2,10 |
| Gelatine 240 Bloom | 8,20 | 9,40 |
| Polydextrose (Litesse^{®} Ultra, Danisco Cultor GmbH) | - | 24,40 |
| Gelber und roter Farbstoff | 0,01 | 0,01 |
| Citronensäure | 0,20 | |
| Kirscharoma, enthaltend 1 Gew.-% | - | 0,10 |
| Extrakt aus Beispiel 2 und 0,3 Gew.-% Phloretin bezogen auf das Aroma | | |

Polydextrose ist ein selbst nicht süß schmeckendes Polysaccharid mit niedrigem Brennwert.

### Anwendungsbeispiel 20: Schoko-Cappuccino-Eiscreme

| | **Zubereitung (Gehalt in Gew.-%)** | |
|---|---|---|
| **Inhaltsstoff** | **A** | **B** |
| Glucose-Fructose-Sirup | 14,10 | 14,10 |
| Saccharose | 10,00 | 7,50 |
| Magermilchpulver | 5,00 | 5,00 |
| Sahne (36% Fettanteil) | 24,00 | 24,00 |
| Emulgator und Stabilisator Cremodan^{®} 709VEG (Danisco) | 0,50 | 0,50 |
| Kakaopulver | 5,975 | 5,975 |
| Carrageenan | 0,025 | 0,025 |
| Wasser | 40,20 | 42,50 |
| Cappuccino-Aroma | 0,20 | 0,20 |
| enthaltend 1 Gew.-% Extrakt aus Beispiel 2 und 1 Gew.-% Homoeriodictyol-Natriumsalz bezogen auf das Aroma | - | 0,20 |

### Anwendungsbeispiel 21: Gelatinekapseln zum Direktverzehr

| | **Zubereitung (Gehalt in Gew.-%)** | | |
|---|---|---|---|
| **Inhaltsstoff** | **A** | **B** | **C** |
| **Gelatinehülle:** | | | |
| Glycerin | 2,014 | 2,014 | 2,014 |
| Gelatine 240 Bloom | 7,91 | 7,91 | 7,91 |
| Sucralose | 0,065 | 0,065 | 0,065 |
| Allura Rot | 0,006 | 0,006 | 0,006 |
| Brillantblau | 0,005 | 0,005 | 0,005 |
| **Kernzusammensetzung:** | | | |
| Pflanzenöl-Triglycerid (Kokosöl - Fraktion) | 79,55 | 68,70 | 58,95 |
| Orangen-Aroma, enthaltend 1 Gew.-% Extrakt aus Beispiel 2 und 1 Gew.-% Homoeriodictyol-Natriumsalz bezogen auf das Aroma | 10,0 | 20,0 | 28,65 |
| Rebaudiosid A 98 % | 0,05 | 0,05 | - |
| 2-Hydroxypropylmenthylcarbonat | 0,33 | 0,20 | - |
| 2-Hydroxyethylmenthylcarbonat | - | 0,20 | 1,00 |
| (1R,3R,4S) Menthyl-3-carbonsäure-N-ethylamid (WS-3) | - | 0,55 | 0,50 |
| (-)-Menthonglycerinacetal (Frescolat MGA) | - | 0,30 | 0,80 |
| Vanillin | 0,07 | - | 0,10 |

Die zum Direktverzehr geeigneten Gelatinekapseln wurden gemäß WO 2004/050069 hergestellt und hatten einen Durchmesser von 5 mm, das Gewichtsverhältnis von Kernmaterial zu Hüllmaterial lag bei 90 : 10. Die Kapseln öffneten sich im Mund innerhalb von weniger als 10 Sekunden und lösten sich vollständig innerhalb von weniger als 50 Sekunden auf.

## Patentansprüche

1. Aromamischung, umfassend
(i) einen oder mehrere süß schmeckende Stoffe, ausgewählt aus der Gruppe der natürlich vorkommenden süß schmeckenden Stoffe und deren physiologisch verträglichen Salzen ohne Phyllodulcin und dessen physiologisch verträglichen Salzen,
und
(ii) Phyllodulcin und/oder eines oder mehrere dessen physiologisch verträgliche Salze, **dadurch gekennzeichnet, dass** das Verhältnis des Sucroseäquivalenz der Konzentration des Stoffes oder der Stoffe der Gruppe (i) zur Sucroseäquivalenz der Konzentration des Stoffes oder der Stoffe der Gruppe (ii) ≥ 2 ist.

2. Aromamischung nach Anspruch 1, umfassend einen Extrakt aus *Hydrangea du*/*cis* als Quelle für die Verbindung oder die Verbindungen der Gruppe (ii).

3. Aromamischung nach Anspruch 1 oder 2, umfassend einen Extrakt aus *Hydrangea macrophylla* var. thunbergii, *Hydrangea macrophylla* var. oamacha oder *Hydrangea macrophylla var.* Amagiana.

4. Aromamischung nach einem der vorangehenden Ansprüche, umfassend darüber hinaus
(iii) einen oder mehrere Stoffe, ausgewählt aus der Gruppe bestehend aus einen süßen Geschmack verstärkenden Aroma- und/oder Geschmackstoffen und deren physiologisch verträglichen Salzen.

5. Aromamischung nach einem der vorangehenden Ansprüche, wobei der oder die Stoffe der Gruppe (i) ausgewählt sind aus Gruppe bestehend aus:
a) Kohlenhydraten ausgewählt aus der Untergruppe bestehend aus Sucrose, Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd und Maltodextrin;
b) natürlich vorkommenden Zuckeralkoholen ausgewählt aus der Untergruppe bestehend aus Glycerin, Erythritol, Threitol, Arabitol, Ribitol, Xylitol, Sorbitol, Mannitol, Maltitol, Isomaltit, Dulcitol und Lactitol;
c) natürlich vorkommenden Süßstoffen, ausgewählt aus der Untergruppe bestehend aus Miraculin, Curculin, Monellin, Mabinlin, Thaumatin, Curculin, Brazzein, Pentadin, D-Phenylalanin und D-Tryptophan;
d) natürlich vorkommenden Süßstoffen, ausgewählt aus der Untergruppe bestehend aus Steviosid, Steviolbiosid, Rebaudiosid A, weiteren Steviolglycosiden wie Rebaudiosid B, Rebaudiosid C, Rebaudiosid D, Rebaudiosid E, Rebaudiosid F, Rebaudiosid G, Rebaudiosid H, Dulcosid und/oder Rubusosid, Oslandin, Polypodosid A, Strogin 1, Strogin 2, Strogin 4, Selligueanin A, Dihydroquercetin-3-acetat, Perillartin, Telosmosid A₁₅, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Camosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Hemandulcin und Glycyrrhetinsäure;
e) den physiologisch verträglichen, süß schmeckenden Derivaten der Mitglieder der Untergruppen a) bis d)
und
f) den physiologisch verträglichen Salzen der Mitglieder der Untergruppen a) bis e), insbesondere deren Kalium-, Natrium-, Calcium- oder Ammoniumsalzen.

6. Aromamischung nach Anspruch 4 oder 5, wobei der oder die Stoffe der Gruppe (iii) ausgewählt sind aus Gruppe bestehend aus
- Hesperetin
- Phloretin
- 3',7-Dihydroxy-4'-methoxyflavan und
- (S)-3',7-Dihydroxy-4'-methoxyflavan.

7. Aromamischung nach einem der vorangehenden Ansprüche, umfassend einen oder mehrere weitere Aroma-, Hilfs- oder Trägerstoffe.

8. Oral konsumierbares Produkt, umfassend eine Aromamischung nach einem der vorangehenden Ansprüche, **dadurch gekennzeichnet, dass** das Verhältnis der Sucroseäquivalenz der Konzentration des Stoffes oder der Stoffe der Gruppe (i) zur Sucroseäqukalenz der Konzentration des Stoffes oder der Stoffe der Gruppe (ii) ≥ 2 ist und
gegebenenfalls einen oder mehrere weitere übliche Lebensmittelbestandteile.

9. Oral konsumierbares Produkt nach Anspruch 8, wobei die Konzentration der Aromamischung so eingestellt ist, dass die Konzentration der Stoffe der Gruppe (ii) 0,5 bis 15 ppm beträgt, bezogen auf des Gewicht des Produktes.

10. Oral konsumierbares Produkt nach Anspruch 8 oder 9, wobei die Konzentration der Aromamischung so eingestellt ist, dass die Konzentration der Stoffe der Gruppe (i) alleine eine Sucroseäquivalenz einer Lösung von ≥ 4 Gew.-% bevorzugt ≥ 6 Gew.-% Sucrose in Wasser besitzt.

11. Oral konsumierbares Produkt nach einem der Ansprüche 8 bis 10, ausgewählt aus der Gruppe bestehend aus pharmazeutischer Zubereitung, der Mundpflege dienende Zubereitung, Halbfertigware, flüssigem und festem Lebensmittel.

12. Verfahren zur Herstellung eines süßmittelreduzierten oral konsumierbaren Produktes, umfassend die Schritte:
a) Bereitstellen einer Aromamischung nach einem der Ansprüche 1 bis 7 oder der einzelnen Bestandteile einer solchen Aromamischung und
b) Einarbeiten der in Schritt a) bereitgestellten Aromamischung oder Bestandteile der Aromamischung in ein oral konsumierbares Produkt, so dass das Verhältnis des Sucroseäquivalenz der Konzentration des Stoffes oder der Stoffe der Gruppe (i) zur Sucroseäquivalenz der Konzentration des Stoffes oder der Stoffe der Gruppe (ii) im fertig hergestellten oral konsumierbaren Produkt ≥ 2 ist.

13. Verfahren nach Anspruch 12, wobei im Schritt b) eine Verdünnung der in Schritt a) bereitgestellten Bestandteile erfolgt, so dass im fertig hergestellten oral konsumierbaren Produkt die Konzentration der Stoffe der Gruppe (ii) 0,5 bis 15 ppm beträgt, bezogen auf des Gewicht des Produktes und/oder die Konzentration der Stoffe der Gruppe (i) alleine eine Sucroseäquivalenz einer Lösung von ≥ 4 Gew.-% bevorzugt ≥ 6 Gew.-% Sucrose in Wasser besitzt.

14. Verwendung einer Aromamischung nach einem der Ansprüche 1 bis 7 zur Herstellung eines süßmittelreduzierten oral konsumierbaren Produktes.

15. Verwendung von Phyllodulcin und/oder eines Extraktes aus *Hydrangea du*/*cis,* umfassend Phyllodulcin und/oder dessen physiologisch verträglichen Salze, zum synergistischen Verstärken des Süßeindruckes einer süß schmeckenden Verbindung.

## Claims

1. Flavouring mixture, comprising
(i) one or more sweet-tasting substances selected from the group of the naturally occurring sweet-tasting substances and their physiologically acceptable salts without phyllodulcin and its physiologically acceptable salts,
and
(ii) phyllodulcin and/or one or more of its physiologically acceptable salts,
**characterised in that** the ratio of the sucrose equivalence of the concentration of the substance or substances of group (i) to the sucrose equivalence of the concentration of the substance or substances of group (ii) is ≥ 2.

2. Flavouring mixture according to claim 1, comprising an extract of *Hydrangea dulcis* as the source of the compound or compounds of group (ii).

3. Flavouring mixture according to claim 1 or 2, comprising an extract of *Hydrangea macrophylla* var. thunbergii, *Hydrangea macrophylla* var. oamacha or *Hydrangea macrophylla* var. Amagiana.

4. Flavouring mixture according to any one of the preceding claims, additionally comprising
(iii) one or more substances selected from the group consisting of aromatic substances and/or flavourings which enhance a sweet taste, and their physiologically acceptable salts.

5. Flavouring mixture according to any one of the preceding claims, wherein the substance or substances of group (i) is/are selected from the group consisting of:
a) carbohydrates selected from the sub-group consisting of sucrose, trehalose, lactose, maltose, melicitose, melibiose, raffinose, palatinose, lactulose, D-fructose, D-glucose, D-galactose, L-rhamnose, D-sorbose, D-mannose, D-tagatose, D-arabinose, L-arabinose, D-ribose, D-glyceraldehyde and maltodextrin;
b) naturally occurring sugar alcohols selected from the sub-group consisting of glycerol, erythritol, threitol, arabitol, ribitol, xylitol, sorbitol, mannitol, maltitol, isomaltitol, dulcitol and lactitol;
c) naturally occurring sweeteners selected from the sub-group consisting of miraculin, curculin, monellin, mabinlin, thaumatin, curculin, brazzein, pentadin, D-phenylalanine and D-tryptophan;
d) naturally occurring sweeteners selected from the sub-group consisting of stevioside, steviolbioside, rebaudioside A, further steviol glycosides such as rebaudioside B, rebaudioside C, rebaudioside D,
rebaudioside E, rebaudioside F, rebaudioside G, rebaudioside H, dulcoside and/or rubusoside, oslandin, polypodoside A, strogin 1, strogin 2, strogin 4, selligueanin A, dihydroquercetin-3-acetate, perillartin, telosmoside A₁₅, periandrin I-V, pterocaryosides, cyclocaryosides, mukuroziosides, trans-anethol, trans-cinnamaldehyde, bryosides, bryonosides, bryonodulcosides, carnosiflosides, scandenosides, gypenosides, trilobatin, phloridzin, dihydroflavanols, hematoxylin, cyanin, chlorogenic acid, albiziasaponine, telosmosides, gaudichaudioside, mogrosides, hernandulcin and glycyrrhetinic acid;
e) the physiologically acceptable, sweet-tasting derivatives of the members of sub-groups a) to d)
and
f) the physiologically acceptable salts of the members of sub-groups a) to e), in particular their potassium, sodium, calcium or ammonium salts.

6. Flavouring mixture according to claim 4 or 5, wherein the substance or substances of group (iii) is/are selected from the group consisting of
- hesperetin,
- phloretin,
- 3',7-dihydroxy-4'-methoxyflavan and
- (S)-3',7-dihydroxy-4'-methoxyflavan.

7. Flavouring mixture according to any one of the preceding claims, comprising one or more further aromatic, auxiliary or carrier substances.

8. Orally consumable product comprising a flavouring mixture according to any one of the preceding claims, **characterised in that** the ratio of the sucrose equivalence of the concentration of the substance or substances of group (i) to the sucrose equivalence of the concentration of the substance or substances of group (ii) is ≥ 2, and optionally one or more further conventional foodstuff constituents.

9. Orally consumable product according to claim 8, wherein the concentration of the flavouring mixture is so adjusted that the concentration of the substances of group (ii) is from 0.5 to 15 ppm, based on the weight of the product.

10. Orally consumable product according to claim 8 or 9, wherein the concentration of the flavouring mixture is so adjusted that the concentration of the substances of group (i) alone has a sucrose equivalence of a solution of ≥ 4 wt.%, preferably ≥ 6 wt.%, sucrose in water.

11. Orally consumable product according to any one of claims 8 to 10, selected from the group consisting of pharmaceutical preparation, preparation for oral care, semi-finished product, liquid and solid foodstuff.

12. Process for the production of a reduced-sweetener, orally consumable product, comprising the steps:
a) preparing a flavouring mixture according to any one of claims 1 to 7 or the individual constituents of such a flavouring mixture and
b) incorporating the flavouring mixture or constituents of the flavouring mixture prepared in step a) into an orally consumable product so that the ratio of the sucrose equivalence of the concentration of the substance or substances of group (i) to the sucrose equivalence of the concentration of the substance or substances of group (ii) in the ready-prepared orally consumable product is ≥ 2.

13. Process according to claim 12, wherein in step b) the constituents prepared in step a) are diluted so that, in the ready-prepared orally consumable product, the concentration of the substances of group (ii) is from 0.5 to 15 ppm, based on the weight of the product, and/or the concentration of the substances of group (i) alone has a sucrose equivalence of a solution of ≥ 4 wt.%, preferably ≥ 6 wt.%, sucrose in water.

14. Use of a flavouring mixture according to any one of claims 1 to 7 in the production of a reduced-sweetener, orally consumable product.

15. Use of phyllodulcin and/or of an extract of *Hydrangea dulcis,* comprising phyllodulcin and/or its physiologically acceptable salts, for synergistically enhancing the sweet impression of a sweet-tasting compound.

## Revendications

1. Mélange d'arômes, comprenant
(i) une ou plusieurs substances au goût sucré choisies dans le groupe des substances au goût sucré existant à l'état naturel et leurs sels physiologiquement compatibles sans phyllodulcine et ses sels physiologiquement compatibles,
et
(ii) de la phyllodulcine et/ou un ou plusieurs de ses sels physiologiquement compatibles,
**caractérisé par le fait que** le rapport entre l'équivalence en saccharose de la concentration de la substance ou des substances du groupe (i) et l'équivalence en saccharose de la concentration de la substance ou des substances du groupe (ii) est supérieur ou égal à (≥) 2.

2. Mélange d'arômes selon la revendication 1, comprenant un extrait d'*Hydrangea dulcis* en tant que source du composé ou des composés du groupe (ii).

3. Mélange d'arômes selon la revendication 1 ou 2, comprenant un extrait *d'Hydrangea macrophylla* var. *thunbergii,* d'*Hydrangea macrophylla* var. *oamacha* ou d'*Hydrangea macrophylla* var. *amagiana.*

4. Mélange d'arômes selon l'une des revendications précédentes, comprenant, en outre,
(iii) une ou plusieurs substances choisies dans le groupe constitué de substances aromatiques et/ou gustatives renforçant un goût sucré et leurs sels physiologiquement compatibles.

5. Mélange d'arômes selon l'une des revendications précédentes, la ou les substances du groupe (i) étant choisies dans le groupe constitué:
a) d'hydrates de carbone choisis dans le sous-groupe constitué de saccharose, de tréhalose, de lactose, de maltose, de mélizitose, de mélibiose, de raffinose, de palatinose, de lactulose, de D-fructose, de D-glucose, de D-galactose, de L-rhamnose, de D-sorbose, de D-mannose, de D-tagatose, de D-arabinose, de L-arabinose, de D-ribose, de D-glycéraldéhyde et de la maltodextrine;
b) d'alcools de sucre existant à l'état naturel choisis dans le sous-groupe constitué de la glycérine, de l'érythritol, du thréitol, de l'arabitol, du ribitol, du xylitol, du sorbitol, du mannitol, du maltitol, de l'isomalt, du dulcitol et du lactitol;
c) d'édulcorants existant à l'état naturel choisis dans le sous-groupe constitué de la miraculine, de la curculine, de la monelline, de la mabinline, de la thaumatine, de la curculine, de la brazzéine, de la pentadine, de la D-phénylalaline et du D-tryptophane;
d) d'édulcorants existant à l'état naturel choisis dans le sous-groupe constitué du stévioside, de stéviolbioside, de rébaudioside A, d'autres stéviolglycosides tels que le rébaudioside B, le rébaudioside C, le rébaudioside D, le rébaudioside E, le rébaudioside F, le rébaudioside G, le rébaudioside H, le dulcoside et/ou rubusoside, de l'oslandine, du polypodoside A, de la strogine 1, de la strogine 2, de la strogine 4, de la selliguéanine A, du 3-acétate dihydroquercétine, de la périllartine, du telosmoside A₁₅, de la périandrine I-V, des ptérocaryosides, des cyclocaryosides, des mukuroziosides, du trans-anéthol, du trans-cinnamaldéhyde, des bryosides, des bryonosides, des bryonodulcosides, des camosiflosides, des scandénosides, des gypénosides, de la trilobatine, de la phloridzine, des dihydroflavonols, de l'hématoxyline, de la cyanine, de l'acide chlorogénique, de l'albiziasaponine, des telosmosides, du gaudichaudioside, des mogrosides, de l'hernandulcine et de l'acide glycyrrhétinique;
e) des dérivés à goût sucré physiologiquement compatibles des éléments des sous-groupes a) à d)
et
f) des sels physiologiquement compatibles des éléments des sous-groupes a) à e), notamment leurs sels de potassium, sodium, calcium ou ammonium.

6. Mélange d'arômes selon la revendication 4 ou 5, la ou les substances du groupe (iii) étant choisies dans le groupe constitué
- de l'hespérétine,
- de la phlorétine,
- du 3',7-dihydroxy-4'-méthoxyflavane et
- du (S)-3',7-dihydroxy-4'-méthoxyflavane.

7. Mélange d'arômes selon l'une des revendications précédentes, comprenant une ou plusieurs autres substances aromatiques, auxiliaires ou supports.

8. Produit consommable oralement, comprenant un mélange d'arômes selon l'une des revendications précédentes, **caractérisé par le fait que** le rapport entre l'équivalence en saccharose de la concentration de la substance ou des substances du groupe (i) et l'équivalence en saccharose de la concentration de la substance ou des substances du groupe (ii) est supérieur ou égal à (≥) 2 et,
le cas échéant, un ou plusieurs autres composants habituels des denrées alimentaires.

9. Produit consommable oralement selon la revendication 8, la concentration du mélange d'arômes étant réglée de manière à ce que la concentration des substances du groupe (ii) soit comprise entre 0,5 et 15 ppm, par rapport au poids du produit.

10. Produit consommable oralement selon la revendication 8 ou 9, la concentration du mélange d'arômes étant réglée de manière à ce que la concentration des substances du seul groupe (i) présente une équivalence en saccharose d'une solution contenant au moins 4% en poids, de préférence au moins 6% en poids, de saccharose dans l'eau.

11. Produit consommable oralement selon l'une des revendications 8 à 10, choisi dans le groupe constitué d'une préparation pharmaceutique, d'une préparation de soin buccal, de produits semi-finis, de produits alimentaires liquides et solides.

12. Procédé de fabrication d'un produit consommable oralement à teneur réduite en édulcorant, comprenant les étapes:
a) fourniture d'un mélange d'arômes selon l'une des revendications 1 à 7 ou des constituants individuels d'un tel mélange d'arômes et
b) intégration du mélange d'arômes ou des constituants du mélange d'arômes fournis à l'étape a) dans un produit consommable oralement de manière à ce que le rapport entre l'équivalence en saccharose de la concentration de la substance ou des substances du groupe (i) et l'équivalence en saccharose de la concentration de la substance ou des substances du groupe (ii) dans le produit consommable oralement fabriqué soit supérieur ou égal à (≥) 2.

13. Procédé selon la revendication 12, une dilution des constituants fournis à l'étape a) étant réalisée à l'étape b) de manière à ce que la concentration des substances du groupe (ii) dans le produit consommable oralement fabriqué soit comprise entre 0,5 et 15 ppm, par rapport au poids du produit, et/ou à ce que la concentration des substances du seul groupe (i) présente une équivalence en saccharose d'une solution contenant au moins 4% en poids, de préférence au moins 6% en poids, de saccharose dans l'eau.

14. Utilisation d'un mélange d'arômes selon l'une des revendications 1 à 7 pour la fabrication d'un produit consommable oralement à teneur réduite en édulcorant.

15. Utilisation de phyllodulcine et/ou d'un extrait d'*Hydrangea dulcis* comprenant de la phyllodulcine et/ou ses sels physiologiquement compatibles pour le renforcement synergique de la saveur sucrée d'un composé au goût sucré.
